(19) Europäisches Patentamt
European Patent Office
Office européen des brevets

(11) EP 1 409 476 B1

(12) EUROPEAN PATENT SPECIFICATION

(45) Date of publication and mention of the grant of the patent: **15.12.2004 Bulletin 2004/51**

(21) Application number: **02746452.8**

(22) Date of filing: **27.06.2002**

(51) Int Cl.[7]: **C07D 333/60**, A61K 31/38, A61P 25/00, C07D 495/04 // (C07D495/04, 333:00), C07D311:00

(86) International application number: **PCT/US2002/016594**

(87) International publication number: **WO 2003/006455 (23.01.2003 Gazette 2003/04)**

# (54) PHARMACEUTICAL COMPOUNDS WITH SEROTONIN RECEPTOR ACTIVITY

PHARMAZEUTISCHE VERBINDUNGEN MIT SEROTONIN REZEPTOR AKTIVITÄT

COMPOSES PHARMACEUTIQUES A ACTIVITE DE RECEPTEUR DE SEROTONINE

(84) Designated Contracting States:
**AT BE CH CY DE DK ES FI FR GB GR IE IT LI LU MC NL PT SE TR**

(30) Priority: **11.07.2001 US 361611 P**
**11.07.2001 EP 01500181**

(43) Date of publication of application: **21.04.2004 Bulletin 2004/17**

(73) Proprietor: **ELI LILLY AND COMPANY**
**Indianapolis, Indiana 46285 (US)**

(72) Inventors:
- **GILMORE, Jeremy Eli Lilly and Company Limited Basingstoke, Hampshire RG21 2XA (GB)**
- **LAMAS-PETEIRA, Carlos Lilly S. A. 28100 Alcobendas (ES)**
- **TORRADO VARELA, Maria, Alicia Lilly S. A. 28020 Alcobendas (ES)**

(74) Representative: **Hiscock, Ian James**
**European Patent Operations,**
**Eli Lilly and Company Limited,**
**Lilly Research Centre,**
**Erl Wood Manor,**
**Sunninghill Road**
**Windlesham GU20 6PH (GB)**

(56) References cited:

| | |
|---|---|
| **WO-A-00/40581** | **WO-A-99/55694** |
| **WO-A-99/55695** | **US-A- 6 162 803** |

- **BERARDI F ET AL: "novel potent s1 ligands: N-[w-(tetralin-1-yl)alkyl]piperidine derivatives" JOURNAL OF MEDICINAL CHEMISTRY, USA, vol. 39, 1996, pages 4255-4260, XP002218227**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 409 476 B1

## Description

This invention relates to novel compounds, their preparation and use as pharmaceuticals.

The compounds of the invention are of the following general formula:

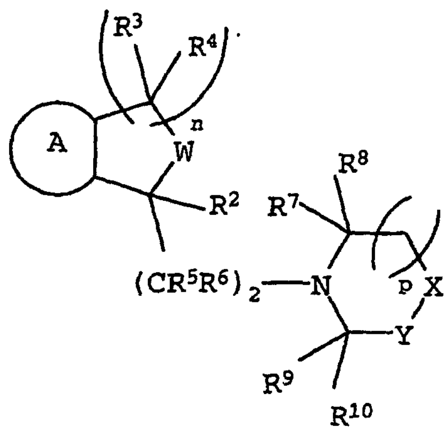

**(I)**

in which

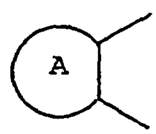

is

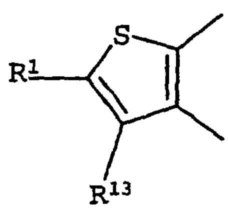

or

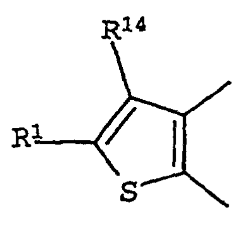

$R^1$ is -CN, $-CONR^{11}R^{12}$, $-COOR^{21}$,

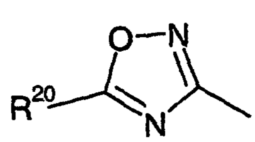

,

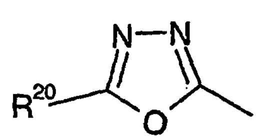

,

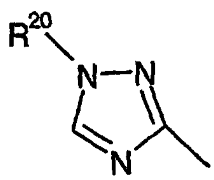, 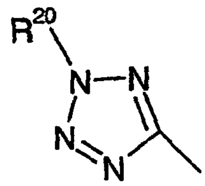, 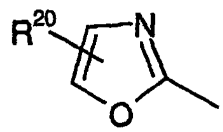

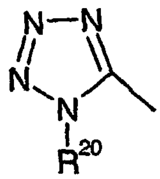, 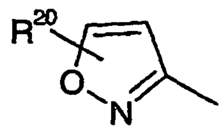 or 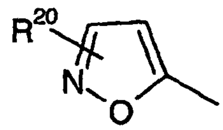

where $R^{11}$ and $R^{12}$ are each hydrogen or $C_{1-6}$ alkyl, or $R^{11}$ and $R^{12}$ taken together with the nitrogen atom to which they are attached form a morpholino, pyrrolidino or piperidinyl ring optionally substituted with one or two $C_{1-6}$ alkyl groups;
$R^2$, $R^3$, $R^4$, $R^5$, $R^6$, $R^7$, $R^8$, $R^9$, $R^{10}$, $R^{20}$ and $R^{21}$ are each hydrogen or $C_{1-6}$ alkyl;
$R^{13}$ and $R^{14}$ are each independently selected from hydrogen, $C_{1-6}$ alkyl or halo;
n is 1 or 2;
p is 0, 1 or 2;
-W- is -O- or $-CF_2-$;
-X-Y- is

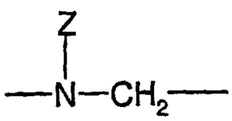, 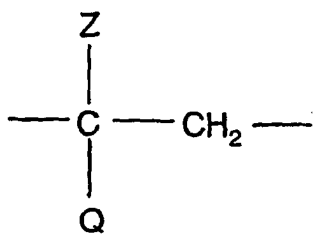 or 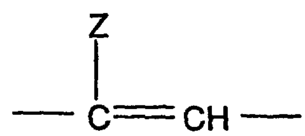

where Z is

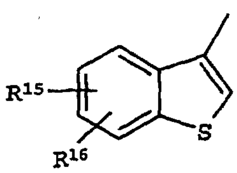
(i)

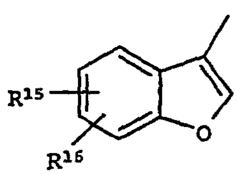
(ii)

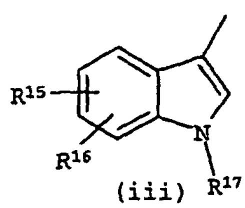
(iii)

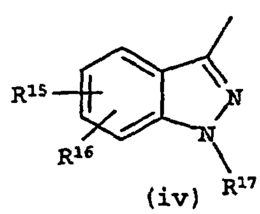
(iv)

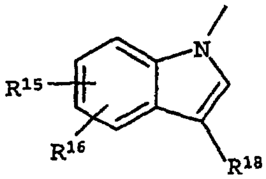
(v)

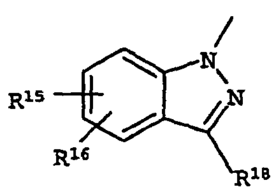
(vi)

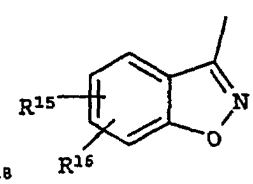
(vii)

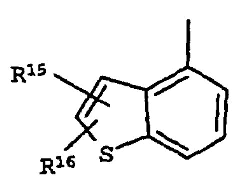
(viii)

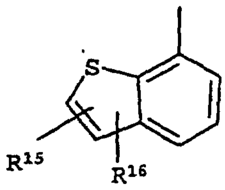
(ix)

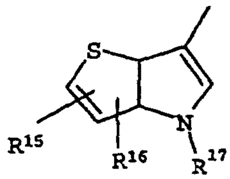
(x)

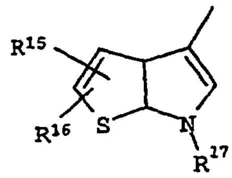
(xi)

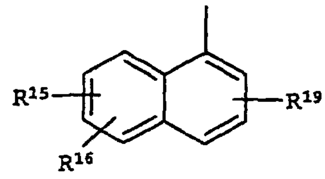
(xii)

where $R^{15}$, $R^{16}$ and $R^{19}$ are each hydrogen, halo, $C_{1-6}$ alkyl or $C_{1-6}$ alkoxy, $C_{1-6}$ alkoxycarbonyl, cyano, trifluoromethyl, trifluoromethoxy, nitro, amino, $C_{1-6}$ acylamino and $C_{1-6}$ alkylthio; and
$R^{17}$ and $R^{18}$ are hydrogen or $C_{1-6}$ alkyl;
Q is hydrogen, halo, nitrile, $C_{1-6}$ alkoxycarbonyl, hydroxy, $C_{1-6}$ alkyl or $C_{1-6}$ alkoxy; and pharmaceutically acceptable salts thereof.

The compounds of the invention and their pharmaceutically acceptable salts are indicated for use in the treatment of disorders of the central nervous system.

In the above formula (I), a $C_{1-6}$ alkyl group can be branched or unbranched and, for example, includes methyl, ethyl, propyl, isopropyl, butyl, isobutyl, t-butyl, pentyl and hexyl, and is preferably methyl or ethyl, and especially methyl.

A $C_{1-6}$ alkoxy group is one such alkyl group linked to a ring through an oxygen atom, and is preferably methoxy or ethoxy, and especially methoxy.

A $C_{1-6}$ alkylthio is an alkyl group linked to a sulphur atom, where the alkyl is as defined above. A $C_{1-6}$ alkylthio group includes for example thiomethyl or thioethyl.

A $C_{1-6}$ acylamino group is an alkyl group linked to an amide group, where the alkyl is as defined above, and is preferably of the formula $R^{IV}$-NH-CO- where $R^{IV}$ is $C_{1-5}$ alkyl. A $C_{1-6}$ acylamino group includes for example acetamide.

A $C_{1-6}$ alkoxycarbonyl is an alkyl chain linked to a group of the formula -OCO-, where the alkyl is as defined above. A $C_{1-6}$ alkoxycarbonyl group includes for example methoxycarbonyl or ethoxycarbonyl.

A halo group is preferably fluoro, chloro or bromo, and especially fluoro.

When n is 2 it will be appreciated that the values of $R^3$ and $R^4$ in the repeated units can be different.

Preferred compounds of the invention have one or more of the following features:

1) n is 2.
2) $R^1$ is -CN.
3) $R^1$ is -COOR$^{21}$.
4) $R^1$ is -CONR$^{11}$R$^{12}$.
5) $R^{21}$ is $C_{1-6}$ alkyl.
6) $R^{21}$ is methyl
7) $R^{11}$ and $R^{12}$ are both hydrogen.
8)

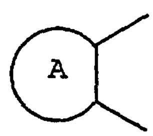

is

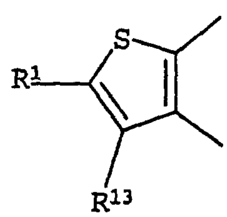

9)

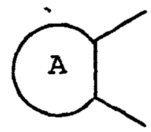

is

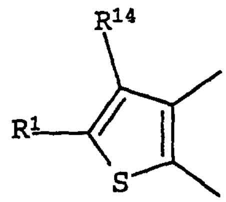

10) $R^{13}$ is hydrogen.
11) $R^{14}$ is hydrogen.
12) $R^{14}$ is halo, especially fluoro or chloro.
13) $R^{14}$ is $C_{1-6}$ alkyl, especially methyl.
14) $R^2$ is hydrogen or $C_{1-6}$ alkyl.
15) $R^2$ is hydrogen or methyl.
16) $R^2$ is hydrogen.
17) $R^3$ to $R^6$ are each hydrogen.
18) $R^7$ to $R^{10}$ are each $C_{1-6}$ alkyl, especially methyl or ethyl, or hydrogen.
19) One of $R^7$ and $R^8$ or one of $R^9$ and $R^{10}$ is $C_{1-6}$ alkyl and the remainder are each hydrogen.
20) p is 1.
21) W is -O-.
22) W is $-CF_2-$.
23) -X-Y- is

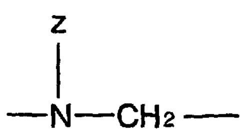

24) -X-Y- is

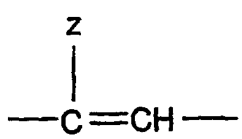

25) Z is

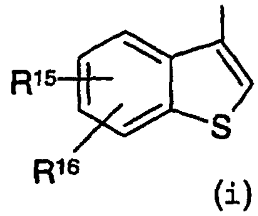

(i)

26) Z is

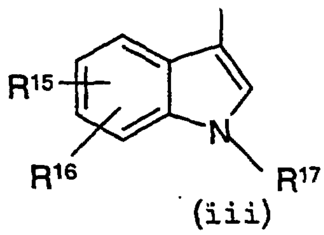

(iii)

27) Z is

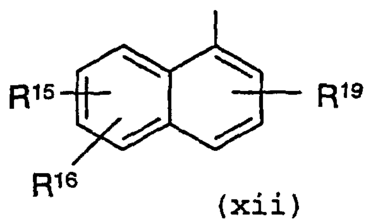

(xii)

28) $R^{15}$, $R^{16}$ and $R^{19}$ are each hydrogen, halo or $C_{1-6}$ alkoxy.
29) One of $R^{15}$, $R^{16}$ and $R^{19}$ is halo or $C_{1-6}$ alkoxy and the remainder are each hydrogen.
30) $R^{15}$ is halo, especially fluoro, $C_{1-6}$ alkoxy, especially methoxy, or hydrogen.
31) $R^{16}$ is halo, especially fluoro, $C_{1-6}$ alkoxy, especially methoxy, or hydrogen.
32) $R^{19}$ is halo, especially fluoro, or hydrogen.
33) $R^{17}$ is hydrogen.

Preferred groups of compounds are those with the following formulas:

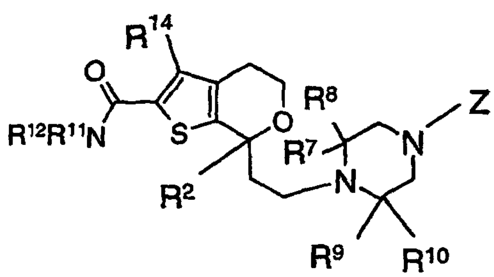

(II)a

wherein:

$R^2$ is methyl or, especially, hydrogen;

One of $R^7$ and $R^8$ or one of $R^9$ and $R^{10}$ is $C_{1-6}$ alkyl and the remainder are each hydrogen;

$R^{11}$ and $R^{12}$ are both hydrogen;

$R^{14}$ is halo, especially fluoro or chloro, $C_{1-6}$ alkyl, especially methyl, or hydrogen;

Z is

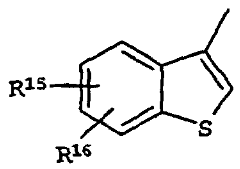
(i)
or
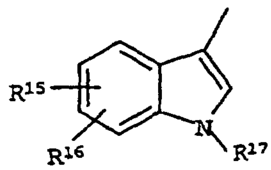
(iii)
or
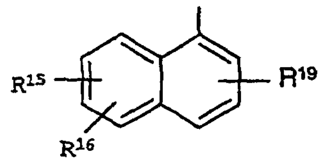
(xii) ,

especially (i) or (xii), where $R^{15}$ , $R^{16}$ and $R^{19}$ are each hydrogen, halo or $C_{1-6}$ alkoxy, and especially, one of $R^{15}$, $R^{16}$ and $R^{19}$ is halo or $C_{1-6}$ alkoxy and the remainder are each hydrogen; and

$R^{17}$ is hydrogen.

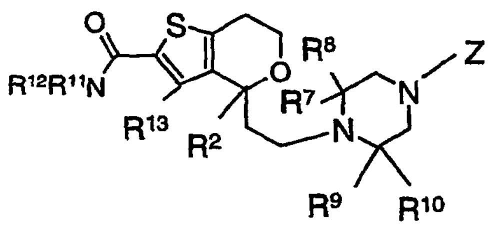

(II)b

wherein:

$R^2$, $R^7$, $R^8$, $R^9$, $R^{10}$, $R^{11}$, and $R^{12}$, are as defined for formula (II)a; Z is as defined for formula (II)a and is especially (xii); and

$R^{13}$ is hydrogen.

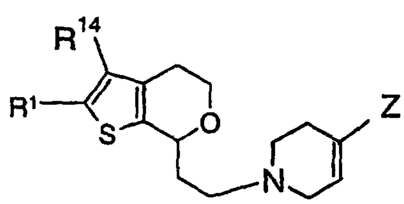

(II)c

wherein:

$R^1$ is -CN, -COOR$^{21}$, where $R^{21}$ is $C_{1-6}$ alkyl, especially methyl, or -CONR$^{11}$R$^{12}$, where both $R^{11}$ and $R^{12}$ are hydrogen;

$R^{14}$ is halo, especially fluoro or chloro, $C_{1-6}$ alkyl, especially methyl, or hydrogen;

Z is

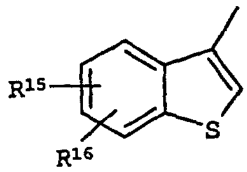

(i)

or

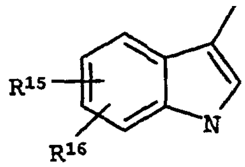

(iii)

or

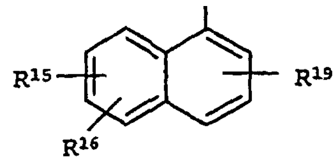

(iii)

especially (iii) or (xii),

where $R^{15}$,$R^{16}$ and $R^{19}$ are each hydrogen, halo or $C_{1-6}$ alkoxy, and especially, one of $R^{15}$ , $R^{16}$ and $R^{19}$ is halo or $C_{1-6}$ alkoxy and the remainder are each hydrogen; and
$R^{17}$ is hydrogen.

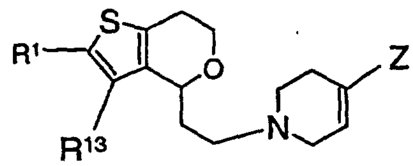

(II)d

wherein:

$R^1$, Z, $R^{15}$, $R^{16}$, $R^{19}$ and $R^{17}$ are as defined in formula (II)c and Z is especially (iii); and
$R^{13}$ is hydrogen.

A particular group of compounds is of the formula:

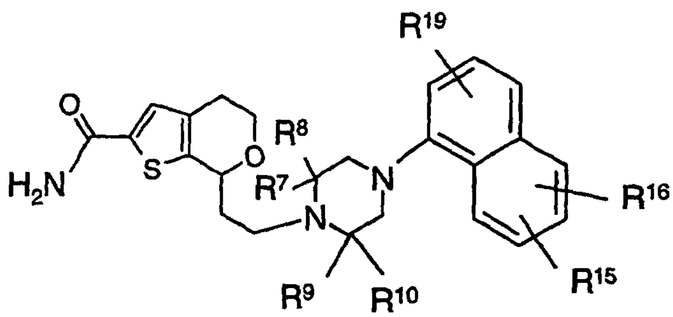

wherein:

One of $R^7$ and $R^8$ or one of $R^9$ and $R^{10}$ is $C_{1-6}$ alkyl and the remainder are each hydrogen;

$R^{15}$, $R^{16}$ and $R^{19}$ are each hydrogen, halo or $C_{1-6}$ alkoxy, and especially, one of $R^{15}$, $R^{16}$ and $R^{19}$ is halo or $C_{1-6}$ alkoxy and the remainder are each hydrogen.

As indicated above, it is, of course, possible to prepare salts of the compounds of the invention and such salts are included in the invention. Acid addition salts are preferably the pharmaceutically acceptable, non-toxic addition salts with suitable acids, such as those with inorganic acids, for example hydrochloric, hydrobromic, nitric, sulphuric or phosphoric acids, or with organic acids, such as organic carboxylic acids, for example glycollic, maleic, hydroxymaleic, fumaric, malic, tartaric, citric, salicyclic, o-acetoxybenzoic, or organic sulphonic, 2-hydroxyethane sulphonic, toluene-p-sulphonic, naphthalene-2-sulphonic or bisethane sulphonic acids. The phosphate is a most preferred salt.

In addition to the pharmaceutically acceptable salts, other salts are included in the invention. They may serve as intermediates in the purification of compounds or in the preparation of compounds or in the preparation of other, for example pharmaceutically acceptable acid addition salts, or are useful for identification, characterisation or purification.

It will be appreciated that the compounds of the invention can contain one or more asymmetric carbon atoms which gives rise to isomers. The compounds are normally prepared as racemic mixtures, but individual isomers can be isolated by conventional techniques if so desired. Such racemic mixtures and individual optical isomers form part of the present invention, the compounds being employed as racemates or in enantiomerically pure form.

The compounds of the invention can be produced by reacting a compound having the formula:

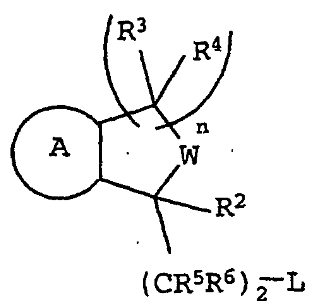

(III)

where L is a leaving group, with a compound of the formula:

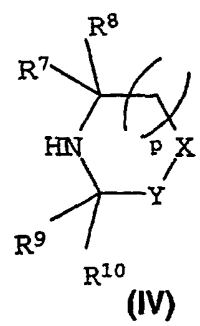

(IV)

where the substituents have the values defined for formula (I) above.

The reaction is preferably carried out in the presence of a base such as potassium carbonate, in an organic solvent such as a polar aprotic solvent, for example, acetonitrile, at a temperature of from 20°C to 100°C. Examples of suitable leaving groups are mesylate, tosylate, triflate, chloride, bromide and iodide.

Intermediate compounds of formula (III) can, for example, be prepared from the corresponding alcohols of the formula:

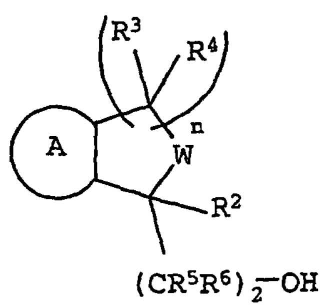

(V)

using standard methods known in the literature such as the ones shown in March, Advanced Organic Chemistry, Fourth Edition, for example the methods mentioned on pages 353 and 354.

Compounds of formula (IV) can be prepared by a variety of methods well known in the art. Substituted 3-(1,2,3,6-tetrahydro-4-pyridinyl)-1*H*-indoles were prepared using methods described in European patent application

1999 EP 897921 and WO patents 9958525 and 002341; fluoro substituted-3-(4-piperidinyl)-1H-indoles and (3R)-6-fluoro-3-(3-pyrrolidinyl)-1H-indole may also be prepared by methods described in these applications. Substituted and unsubstituted 4-(1-naphthyl)-1,2,3,6-tetrahydropyridines and 4-(1-naphthyl)piperidines may be prepared using methods described in USA patents 5,472,966, 5,250,544, and 5,292,711. Substituted and unsubstituted 1-(1-naphthyl) piperazines may be prepared using methods described in USA patent 5,166,156. (2*R*,4*S*)-2-methyl-4-(2-naphthyl) piperidine may be prepared using methods referred to in *Med.Chem.* Res. (1997), 7(4), 207-218. Substituted and unsubstituted 4-(2-benzopyran-3-yl)-1,2,3,6-tetrahydropyridines and 4-(1-benzopyran-3-yl)piperidines may be prepared using methods described in Eur. Pat. Appl. (1992) EP 466585 or in Japanese patent JP 2000086603. 6-fluoro-3-(1,2,3,6-tetrahydro-4-pyridinyl)-1,2-benzisoxazole may be prepared using methods based on USA patent US 3678062. Substituted and unsubstituted 6-fluoro-1-3-(1,2,3,6-tetrahydro-4-pyridinyl)-1*H*-indazoles may be prepared by methods described in EP 135781 (1985). 4-(Thieno[3,2-b]pyrrol-6-yl)-1,2,3,6-tetrahydropyridine may be prepared by methods found in *Heterocycl. Commun.* (1999), 5(4) 305-310. Substituted and unsubstituted 4-(1-benzothieny-7-yl)-1,2,3,6-tetrahydropyridines and 4-(4-fluoro-1-benzopyran-7-yl)-1,2,3,6-tetrahydro-pyridine may be prepared using methods described in WO-0000198. 5-Methoxy-3-(1,2,3,6-tetrahydro-4-pyridinyl)-1*H*-indole may be obtained from Tocris Cookson.

Particular examples of how to prepare intermediates of formula (IV) are explained in more detail below.

For example substituted 4-(7-methoxynaphthalen-1-yl)-1,2,5,6-tetrahydropyridine of formula (IV')can be prepared using scheme (1) below wherein the ketone 1 is converted to the hydrazone and in the presence of a base such as n-butyl lithium and cuprous bromide, undergoes a Shapiro reaction to give the corresponding vinyl bromide 2. Compound 2 is then aromatised using a reagent such as DDQ (2,3-dichloro-5,6-dicyano-1,4-benzoquinone) in a suitable solvent such as toluene. The protected piperidine ring is incorporated using N-protected piperidone such as N-Carbobenzyloxypiperidone in the presence of a suitable base such as n-BuLi in a suitable solvent such as THF. The nitrogen in the piperidine ring is then deprotected using suitable deprotection conditions as those shown in Greene and Wuts, Protecting Groups in Organic Synthesis, 3rd. Ed., John Wiley & Sons. In the particular case wherein CBZ (carbobenzyloxy) is used as a protecting group then hydrogenation conditions can be used in the presence of a suitable catalyst such as Palladium on charcoal in a suitable solvent such as methanol.

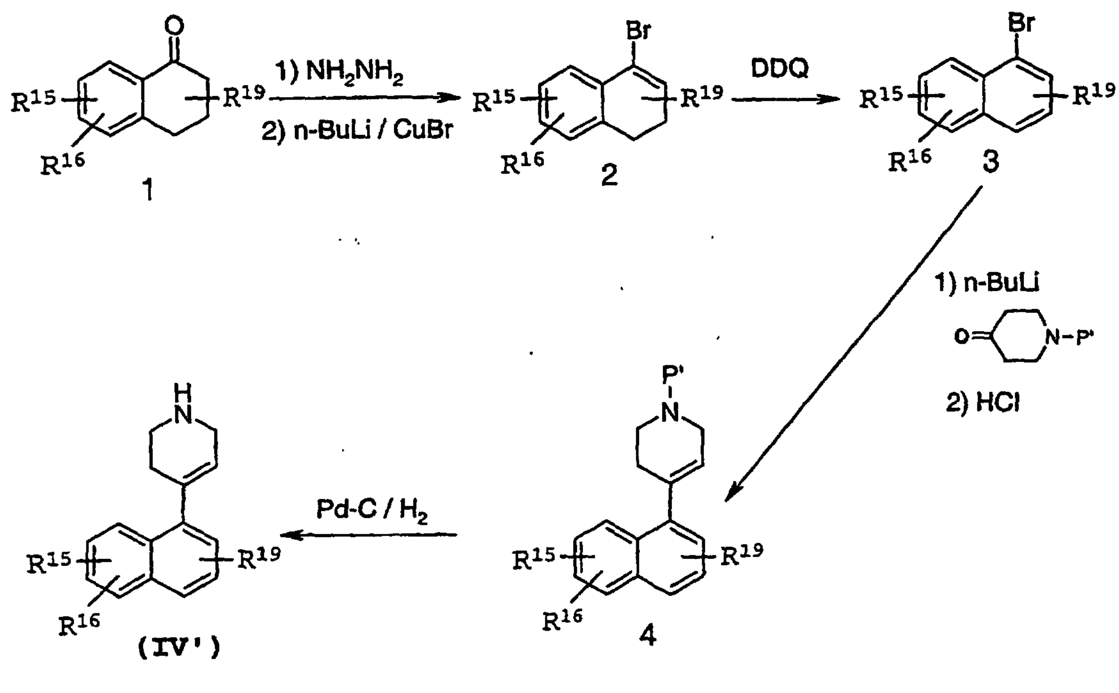


1 2 3 4 (IV')

Scheme (1)

Substituted 1-(naphthalen-1-yl)-3-methylpiperazines of formula (IV")can, for example, be prepared from the corresponding triflate 6. Said triflate can be prepared using methods known in the art such as from the corresponding ketone 5 in the presence of triflic anhydride as shown in scheme (2) below. Compound 6 is then aromatised using a reagent such as DDQ in a suitable solvent such as toluene. Alternatively said triflate 7 can be prepared in one step from the corresponding phenol 8 in the presence of a suitable triflating reagent such as $PhNTf_2$ (N-phenyltrifluoromethanesulfonimide) and a base such as potassium tert-butoxide in a suitable solvent such as THF. The piperazine ring is

then incorporated via palladium catalysed amination of the triflate with 2-methylpiperazine under Hartwig-Buchwald conditions, such as (R)-BINAP, palladium acetate and caesium carbonate in a suitable solvent such as toluene. The nitrogen in the piperazine ring is then deprotected using suitable deprotection conditions as those shown in Greene and Wuts, Protecting Groups in Organic Synthesis, 3rd. Ed., John Wiley & Sons. In the particular case wherein CBZ is used as a protecting group then hydrogenation conditions can be used in the presence of a suitable catalyst such as Palladium on charcoal in a suitable solvent such as methanol.

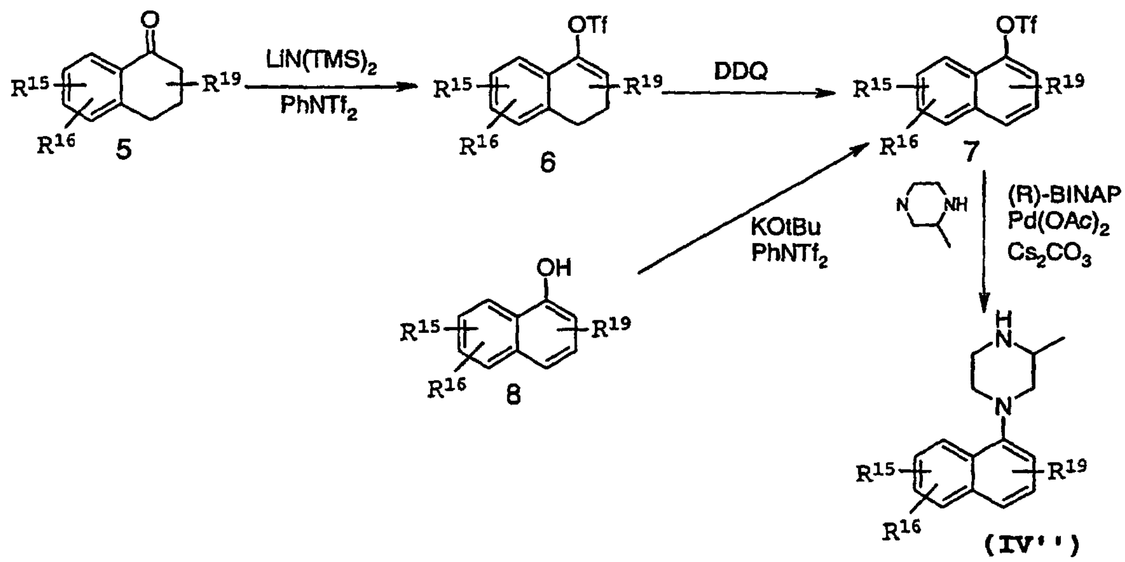


Scheme (2)

Other substituted 1-(naphthalene-2-yl)-3-alkylpiperazines (wherein the alkyl substituent is other than methyl, as for example, ethyl) may be prepared by reacting compounds of formula 7 with the appropriate 2-alkylpiperazine under Hartwig-Buchwald conditions, such as those described in scheme (2).

In general compounds of formula (V) can, for example, be prepared from the appropriate esters of the formula:

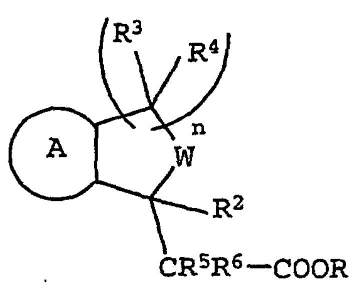

(VI)

where R is $C_{1-6}$ alkyl. Such esters can be reduced in the presence of a reducing agent such as lithium borohydride or lithium aluminium hydride in a suitable organic solvent such as tetrahydrofuran (THF).

Compounds of formula (V) wherein -W- is -O-,

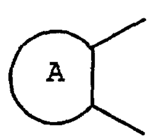

is

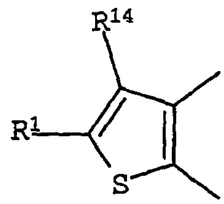

$R^1$ is $-CONR^{11}R^{12}$ and $R^2$ is hydrogen can be prepared from the appropriate 2-(3)-thienylethanols as shown in scheme (3) below:

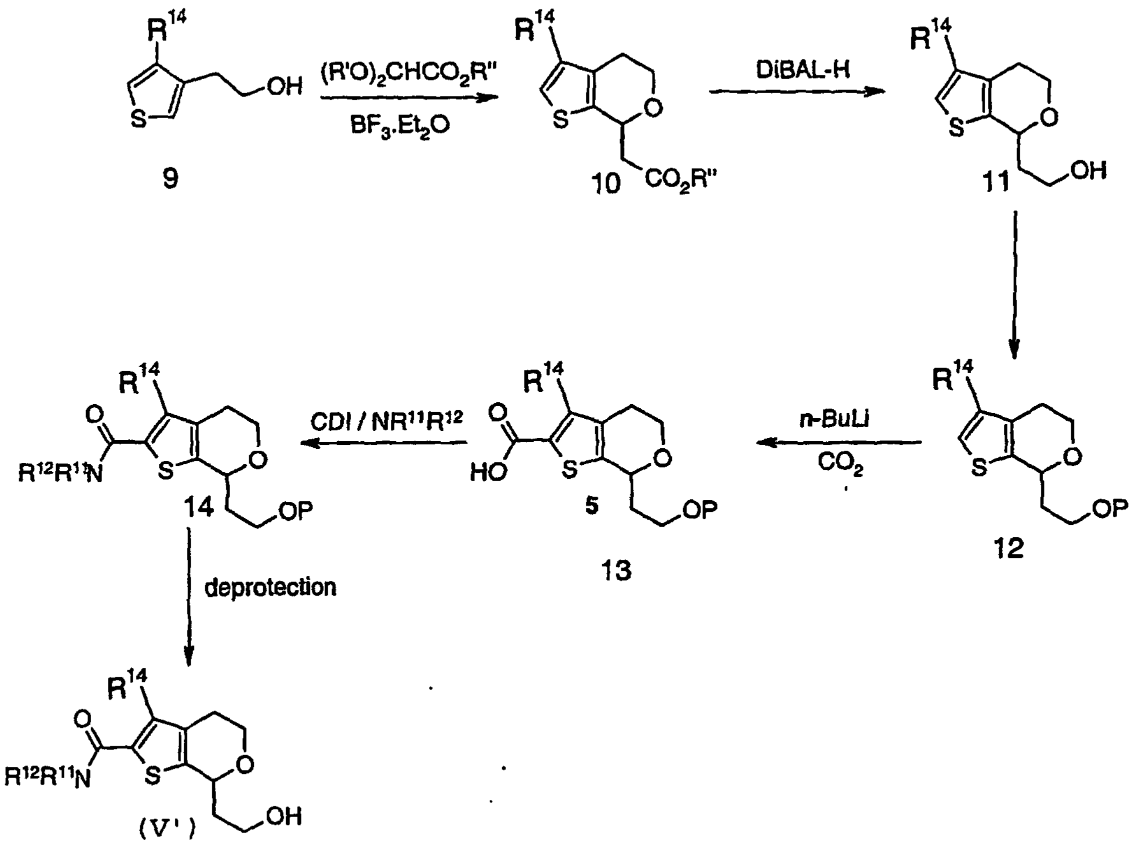


Scheme (3)

wherein R' and R" are each $C_{1-6}$ alkyl groups and P is a suitable alcohol protecting group. Thienylethanols where $R^{14}$ is hydrogen are commercially available and are reacted with appropriate alkyl dialkoxyacetates such as ethyl diethoxyacetate, in a suitable solvent such as dichloromethane, in the presence of a Lewis acid such as borontrifluoride etherate, to give the corresponding alkyl (3-substituted-4,7-dihydro-5*H*-thieno[2,3-c]pyran-7-yl)acetate. The ester is then reduced to the corresponding 2-(3-substituted-4,7-dihydro-5*H*-thieno[2,3-c]pyran-7-yl)ethanol using the conditions described above. Then the alcohol is protected using a suitable protecting group as shown in Greene and Wuts, Protecting Groups in Organic Synthesis, 3rd. Ed., John Wiley & Sons. Preferred protecting groups are silyloxy protecting groups, such as for example tert-butyldimethylsilyl group.

An acid group is then incorporated in the 2 position of the thienopyranyl ring via carboxylation with carbon dioxide and a suitable base such as n-BuLi in a suitable solvent such as tetrahydrofuran. The corresponding carboxy group is then condensed with the appropriate amine of formula $HNR^{11}R^{12}$. Said condensation is preferably carried out in the presence of a coupling reagent such as carbonyldiimidazole (CDI) in a suitable solvent such as dioxan.

The protected alcohol is then deprotected using standard methods known in the literature, such as Greene and Wuts, Protecting Groups in Organic Synthesis, 3rd. Ed., John Wiley & Sons, to give alcohol (V').

Alternatively, the starting thienylethanol 9 can be reacted in the same conditions as shown in scheme (3) above but using the appropriate ketoester $R^2COCH_2CO_2R^{iii}$ to obtain the corresponding alkyl (3-substituted-7-alkyl - 4,7-dihydro-5H-thieno[2,3c]pyran -7-yl)acetate 15 as shown in scheme (4).

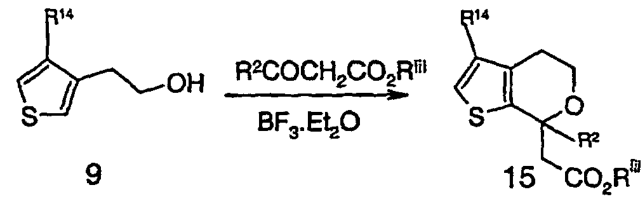

9 15

Scheme (4)

wherein $R^{iii}$ is $C_{1-6}$ alkyl groups and P is a suitable alcohol protecting group. Said intermediate is then derivatised using the synthetic steps shown in scheme (3) and reaction conditions described above to give the corresponding intermediate of formula (V")

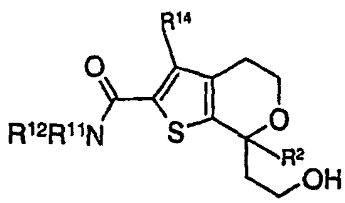

(V'')

It would be appreciated that compounds of formulae (V') and (V") wherein $R^{14}$ is $C_{1-6}$ alkyl can be prepared from the corresponding unsubstituted compounds and then alkylated at some stage during the synthetic process. For example said alkylation can be performed when the 2-(3-substituted-4,7-dihydro-5*H*-thieno[2,3-c]pyran-7-yl)ethanol is protected as shown in the scheme (5) below to give the corresponding alkylated product 17..

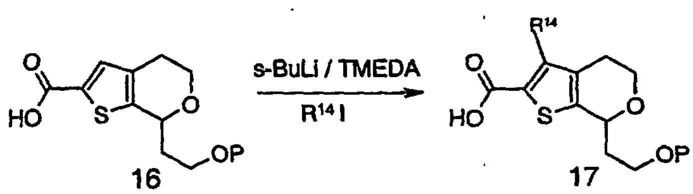

16 17

Scheme (5)

wherein P is a suitable alcohol protecting group. Said intermediate 17 is then derivatised using the synthetic steps shown in scheme (3) and reaction conditions described above to give the corresponding intermediate of formula (V'):

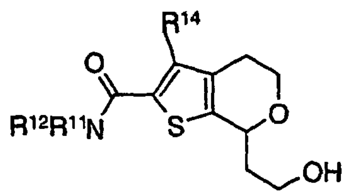

Similarly compounds above wherein $R^{14}$ is a halo group can be prepared as in scheme (5), but replacing the alkyl halide by a suitable halogenating agent, such as for example N-chlorosuccinimide.

Compound of formula (V) wherein

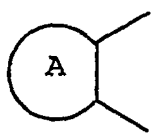

is

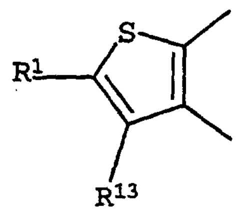

$R^1$ is $-CONR^{11}R^{12}$ and $R^2$ is hydrogen can be prepared from the appropriate 2-(2)-thienylethanols 18 using the same conditions mentioned above as shown in scheme (6) below:

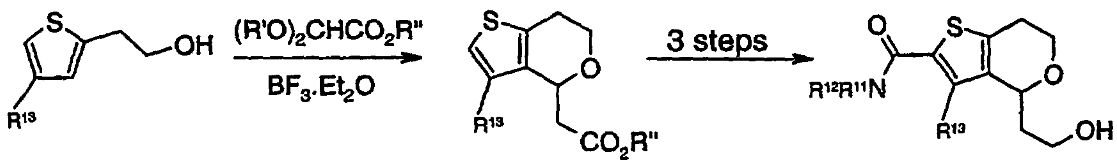


18 19 (V''')

scheme (6)

wherein R' and R" are each $C_{1-6}$ alkyl.

Alternatively, the protected alcohol can be acylated with alkylhaloformates in the presence of a suitable base such as n-BuLi in a suitable solvent such as tetrahydrofuran, as shown in scheme (7) to give the ester intermediate 21, wherein $R^{21}$ is $C_{1-6}$ alkyl and P is a suitable alcohol protecting group.

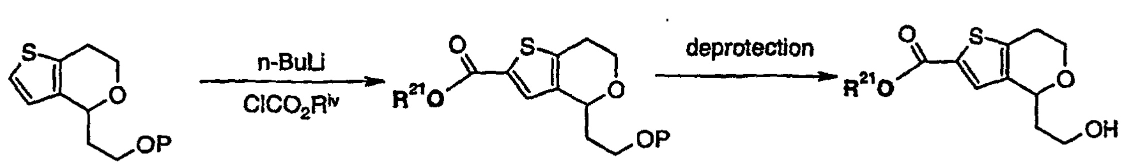


20 21 22

scheme (7)

Compounds of formula (V) wherein

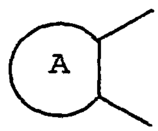

is

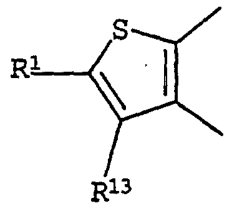

and $R^2$ is $C_{1-6}$ alkyl can be prepared using the appropriate 2-(2-thienyl)ethanol as a starting material. The 2-(2-thienyl) ethanol can be reacted in the same conditions as shown in scheme (4) above to obtain the corresponding alkyl(3-substituted-4-alkyl-6,7-dihydro-4H-thieno[3,2c]pyran-4-yl) acetate 23 shown in scheme (8).

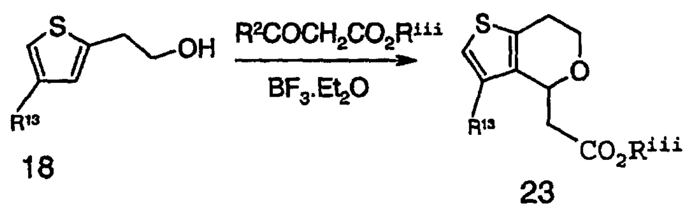


18

23

Scheme (8)

wherein $R^{iii}$ is as defined above. The formula V compound can then be obtained by performing the appropriate steps in scheme (3) above.

Compounds of formula (V) wherein

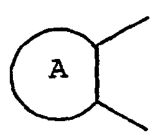

is

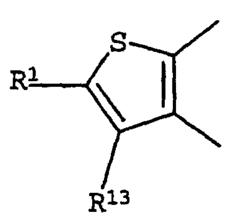

and $R^{13}$ is $C_{1-6}$ alkyl can also be synthesised by following the reaction steps shown in scheme (3), using the appropriate 2-(2-thienyl)ethanol as the starting material. The alkylation reaction required to obtain a formula V compound substituted with a $R^{13}$-alkyl group can be performed, as shown in scheme (9), when the 2-(3-substituted-6,7-dihydro-4H-thieno[3,2c]pyran-4-yl)ethanol is protected.

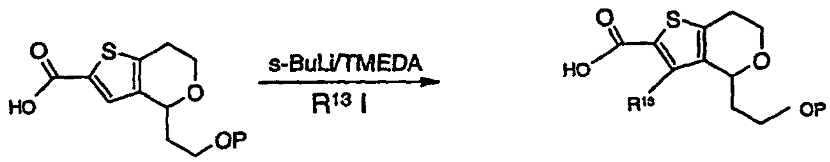


24

25

Scheme (9)

wherein P is as defined above.

Compounds of formula (V) wherein -W- is $-CF_2-$ and $R^1$ is $CONR^{11}R^{12}$ can be prepared from the 3-substituted-5,6-dihydro-1-benzothiophen-7(4*H*)-ones as shown in scheme (10). Said benzothiophen-7(4*H*)-ones are difluorinated in the alpha position to the ketone moiety in the presence of a fluorinating agent, such as $PhSO_2NHF$ and a base, such as $KN(TMS)_2$ in a suitable solvent such as THF.

The resulting ketone 27 is then reacted with activated ylides such as for example a phosphonate of the formula $(R^vO)_2P(O)CH_2CO_2R^{vi}$, wherein $R^v$ and $R^{vi}$ are each $C_{1-6}$ alkyl groups, in the presence of a base such as sodium hydride in a suitable solvent such as for example dioxan to form the corresponding unsaturated ester 28. The ester is reduced as shown and described above in scheme 3 to give the corresponding alcohol 29 which was then protected as before to give intermediate 30 wherein P is a suitable alcohol protecting group. The carboxylation of compound 30 is carried out as before to give intermediate 31. Said alkene 31 is then reduced for example via hydrogenation in the presence of a catalyst such as Pd on charcoal in a suitable solvent such as ethanol or methanol, followed by condensation with an amine $NR^{11}R^{12}$ as before to give intermediate 32. Finally the alcohol is deprotected following suitable conditions as those shown in Greene and Wuts, Protecting Groups in Organic Synthesis, 3rd. Ed., John Wiley & Sons to give alcohol $V^{iv}$.

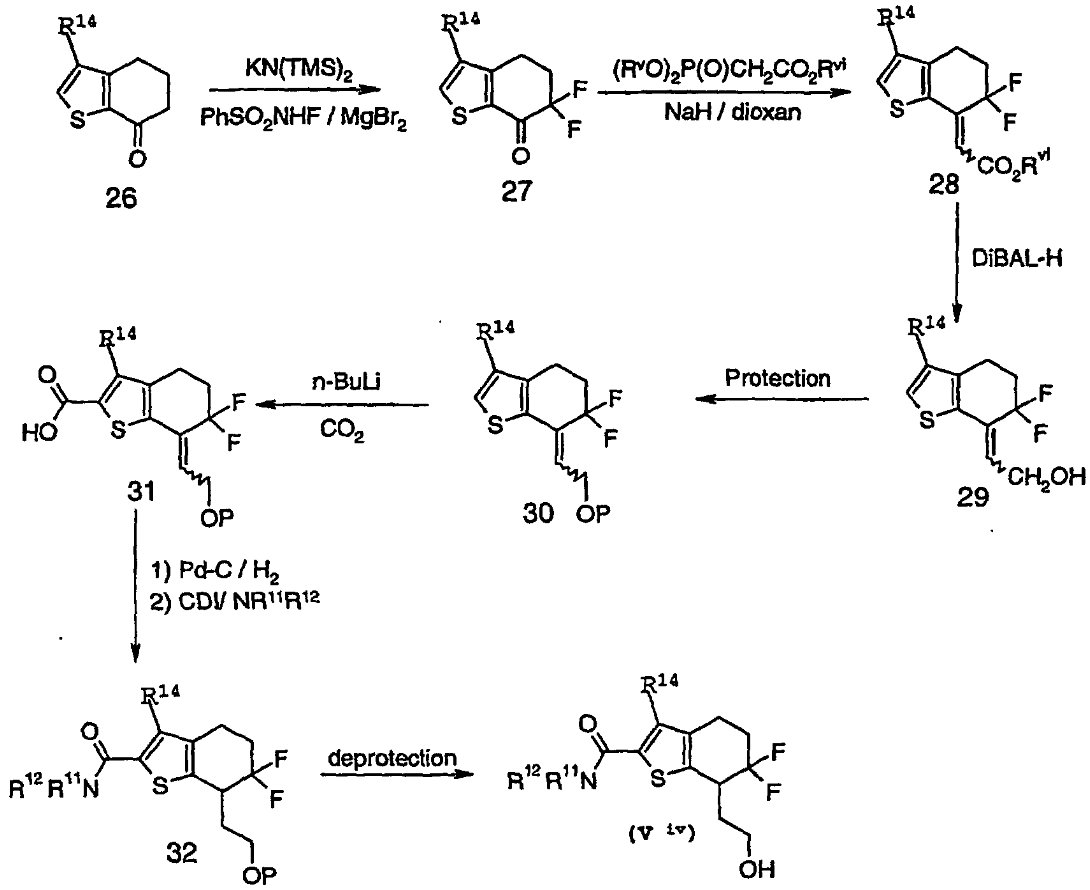


26 27 28 29 30 31 32 (V iv)

Scheme (10)

As before said synthetic route shown in scheme (10) can also be used for 2-(5,5-difluoro-3,4-disubstituted-4,5,6,7-tetrahydro-1-benzothien-4-yl), 2-(5,5-difluoro-3,6-disubstituted-5,6-dihydro-4*H*-cyclopenta[*b*]thien-6-yl) and 2-(5,5-difluoro-3,4-disubstituted-5,6-dihydro-4*H*-cyclopenta[b]thien-4-yl) ethanol derivatives starting from the corresponding 2-substituted-6,7-dihydro-1-benzothiophen-4(5*H*)-one, 3-substituted-4,5-dihydro-6*H*-cyclopenta[*b*]thiophen-6-one and 2-substituted-5,6-dihydro-4*H*-cyclopenta[*b*]thiophen-4-one.

Compounds of formula (I) wherein $R^1$ is

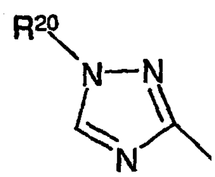

can be synthesised as shown in scheme (11), using the corresponding amide intermediates of formula ($V^v$) wherein the alcohol moiety is protected with an appropriate alcohol protecting group P, such as those shown in Greene and Wuts, Protecting Groups in Organic Synthesis, 3rd. Ed., John Wiley & Sons.

Said intermediates can be formed as shown in schemes (3) to (10) wherein $R^{11}$ and $R^{12}$ are both hydrogen. Amides 33 are cyclised via reaction with dimethylformamide dimethylacetal in a suitable solvent such as toluene, followed by reaction with the corresponding hydrazine of the formula $R^{20}$-NH-$NH_2$ in a suitable solvent such as for example methanol. Then the alcohols are deprotected using methods known in the art such as those shown in Greene and Wuts, Protecting Groups in Organic Synthesis, 3rd. Ed., John Wiley & Sons.

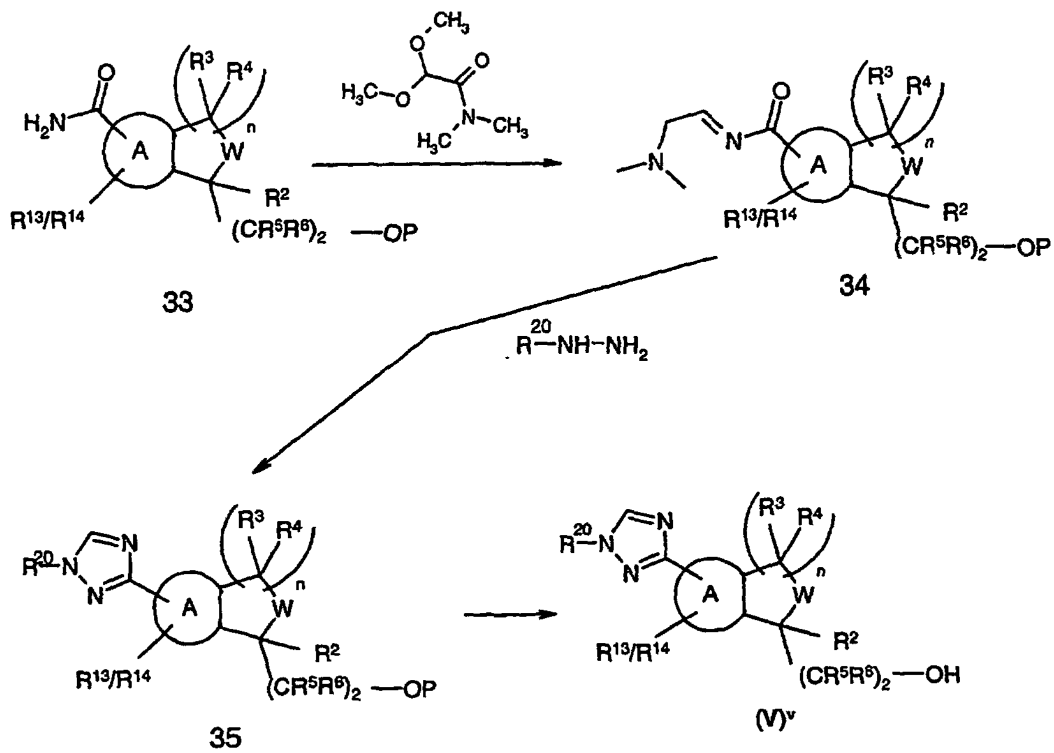


33

34

35

(V)$^v$

Scheme (11)

Compounds of the invention can also be synthesised via reaction of the corresponding amine of the formula 36 with a compound of the formula Z-$L^{iii}$ wherein $L^{iii}$ is a leaving group such as triflate or a halide such as bromide or iodide.

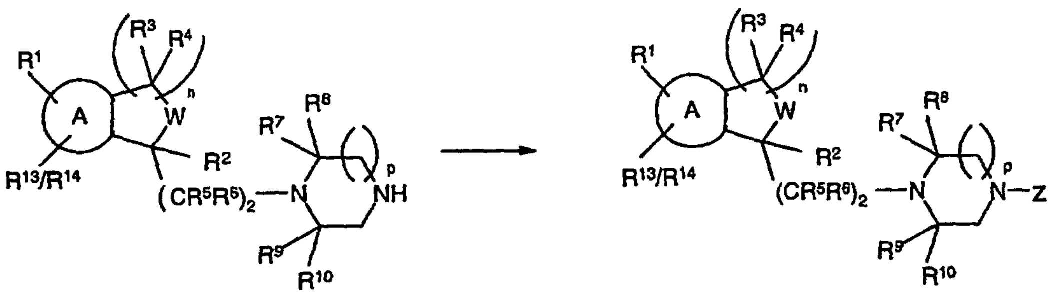

36

(I')

Scheme (12)

Such reactions are usually carried out in the presence of a palladium catalyst and a base such as potassium tertbutoxide.

Some intermediates of the general formula Z-$L^{iii}$ wherein $L^{iii}$ is a halogen group such as bromo are commercially available. Alternatively, they can be synthesised from known literature routes, such as by brominating the corresponding aromatic group with NBS. Intermediates wherein $L^{iii}$ is a triflate can be prepared using methods known in the art such as from the corresponding ketones in the presence of triflic anhydride. Such intermediates are illustrated in

scheme (13) for compound wherein Z is (i) and (xii), but it will appreciated that such method can be used for any values of Z.

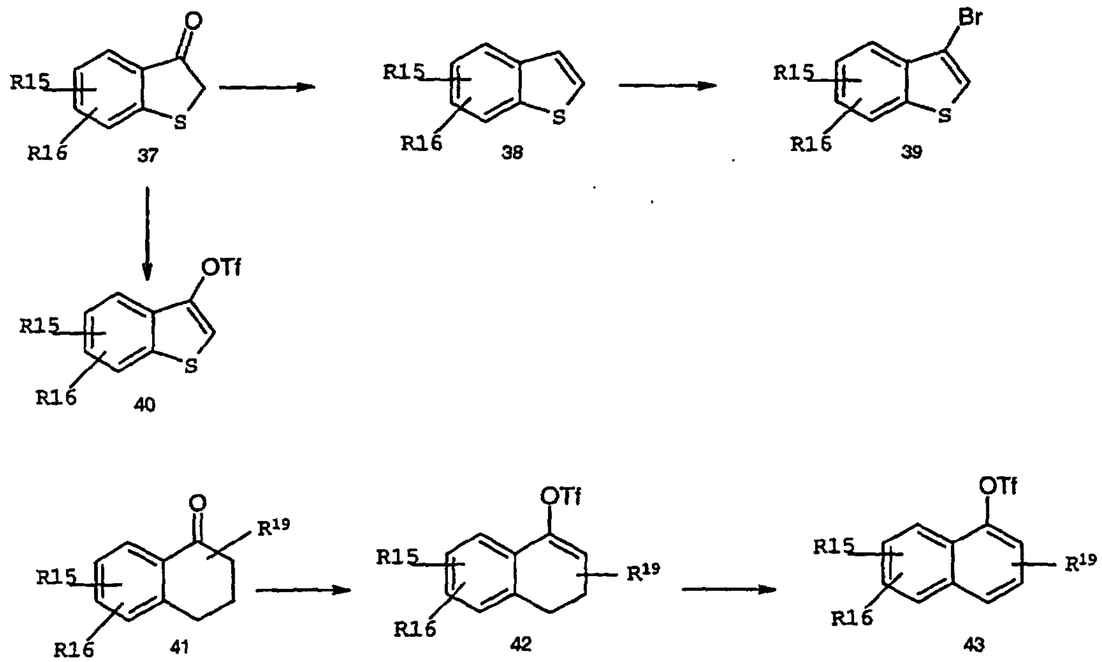


Scheme (13)

It will be appreciated that substituents in the aromatic ring, such as $R^{13}$ and $R^{14}$, may be present in the starting materials or introduced at an appropriate point in the manufacture of the product compound. If necessary said substituents may be protected during the reaction procedure.

Compounds of the invention have been demonstrated to be active at the serotonin, 5-HT 1D receptor. Their binding activity has been demonstrated in a test described by Pullar I.A. *et al*, European Journal of Pharmacology, 407, (2000), 39-40.

As mentioned above, the compounds of the invention and their pharmaceutically acceptable salts have useful central nervous system activity. They have been shown to increase release of tritiated-5HT from guinea pig cortical slices in a test with the following procedure.

Cortical slices from the brains of male guinea pigs were incubated with 50 nM [$^3$H]-5-HT for 30 minutes at 37°C. The slices were washed in basal buffer containing 1 μM paroxetine and then transferred to baskets. The baskets were used to transfer the tissue between the washing and release buffers, all of which contained 1 μM paroxetine.

In order to obtain a stable baseline release, the slices were incubated for 11 minutes in buffer and then transferred for 4 minutes to a second tube containing buffer. Following incubation they were again transferred, for a further 4 minutes, to a buffer in which NaCl had been substituted, on an equimolar basis, to give a KC1 concentration of 30 μM (release sample). The tritium in the tissue samples and in the buffers from the three incubation periods was estimated by liquid scintillation spectroscopy. Test compound was present throughout the three incubation periods. The compounds of the invention enhanced release of 5-HT.

The compounds of the invention are serotonin reuptake inhibitors, and possess excellent activity as, for example, in the test described by Carroll et al., J. Med. Chem. (1993), 36, 2886-2890, in which the intrinsic activity of the compound to competitively inhibit the binding of selective serotonin reuptake inhibitors to the serotonin transporter is measured. These results were also confirmed by *in vivo* tests in which the effect of the compound on a behavioural syndrome in mice dosed with 5-HTP (5-Hydroxytryptophan)and monoamine oxidase inhibitor (MAOI) such as pargyline, is measured, see Christensen, A. V., et al., Eur. J. Pharmacol. 41, 153-162 (1977).

In view of the selective affinity of the compounds of the invention for the serotonin receptors, they are indicated for use in treating a variety of conditions such as depression, bipolar disorder, anxiety, obesity, eating disorders such

as anorexia and bulimia, alcoholism, pain, hypertension, ageing, memory loss, sexual dysfunction, psychotic disorders, schizophrenia, gastrointestinal disorders, headache, cardiovascular disorders, smoking cessation, epilepsy, drug abuse and addiction, emesis, Alzheimer's disease and sleep disorders. The compounds of the invention are principally intended for the treatment of depression or anxiety, or disorders with depressive or anxiety symptoms.

The compounds of the invention are effective over a wide dosage range, the actual dose administered being dependent on such factors as the particular compound being used, the condition being treated and the type and size of animal being treated. However, the dosage required will normally fall within the range of 0.001 to 20, such as 0.01 to 20 mg/kg per day, for example in the treatment of adult humans, dosages of from 0.5 to 100 or 200 mg per day may be used.

The compounds of the invention will normally be administered orally or by injection and, for this purpose, the compounds will usually be utilised in the form of a pharmaceutical composition. Such compositions are prepared in a manner well known in the pharmaceutical art and comprise at least one active compound.

Accordingly the invention includes a pharmaceutical composition comprising as active ingredient a compound of formula (I) or a pharmaceutically acceptable salt thereof, associated with a pharmaceutically acceptable diluent or carrier. In making the compositions of the invention, the active ingredient will usually be mixed with a carrier, or diluted by a carrier, or enclosed within a carrier which may be in the form of a capsule, sachet, paper or other container. More than one active ingredient or excipient may, of course, be employed. The excipient may be a solid, semi-solid or liquid material which acts as a vehicle, excipient or medium for the active ingredient. Some examples of suitable excipients are lactose, dextrose, sucrose, sorbitol, mannitol, starches, gum acacia, calcium phosphate, alginates, tragacanth, gelatin, syrup, methyl cellulose, methyl- and propyl-hydroxybenzoate, talc, magnesium stearate or oil. The compositions of the invention may, if desired, be formulated so as to provide quick, sustained or delayed release of the active ingredient after administration to the patient.

Depending on the route of administration, the foregoing compositions may be formulated as tablets, capsules or suspensions for oral use and injection solutions or suspensions for parenteral use or as suppositories. Preferably the compositions are formulated in a dosage unit form, each dosage containing from 0.5 to 100 mg, more usually 1 to 100 mg, of the active ingredient.

The following Preparations and Examples illustrate routes to the synthesis of the compounds of the invention.

<u>Preparation of 7-(2-hydroxyethyl)-4,5-dihydro-7*H*-thieno[2,3-c]pyran-2-carboxamide</u>

a) Ethyl 4,5-dihydro-7*H*-thieno[2,3-c]pyran-7-yl)acetate

Boron trifluoride etherate (1.17 mmol) was added dropwise at -78ºC to a solution of 2-(3-thienyl)ethanol (1.17 mmol) and ethyl 3,3-diethoxypropionate (1.40 mmol) in dichloromethane (6 mL). The resulting solution was allowed to warm to room temperature overnight. A brine/HC1 mixture (1:1) was added and the aqueous phase was extracted with dichloromethane (3x). The combined organic layers were dried ($MgSO_4$), filtered and evaporated. The crude product was purified by flash column chromatography on silica gel (hexane/EtOAc 9:1) to afford pure ethyl 4,5-dihydro-7*H*-thieno[2,3-c]pyran-7-yl)-acetate. $^1$H-NMR ($CDCl_3$, 300 MHz): δ 1.29 (3H, t, *J* = 7.0 Hz), 2.5-2.7 (1H, m), 2.8 (2H, d, *J* = 6.6 Hz), 2.7-3.0 (1H, m), 3.8 (1H, dt, *J* = 10.7, 4.0 Hz), 4.0-4.2 (1H, m), 4.22 (2H, c, *J* = 7.0 Hz), 5.27 (1H, t, *J* = 7.0 Hz), 6.81 (1H, d, *J* = 5.0 Hz), 7.15 (1H, d, *J* = 5.0 Hz) ppm.

b) 2-(4,5-Dihydro-7*H*-thieno[2,3-c]pyran-7-yl)ethanol

To a solution of ethyl 4,5-dihydro-7*H*-thieno[2,3-c]pyran-7-yl)-acetate (4.86 mmol) in tetrahydrofuran was slowly added, DiBAL-*H* (11.67 mmol) at 0ºC and the mixture stirred for 1h at room temperature before being quenched by careful addition of hydrochloric acid (3N) at 0ºC. The resulting mixture was filtered through Celite, the phases separated and the aqueous phase extracted with dichloromethane (3x). The combined organic phases were dried ($MgSO_4$), filtered and evaporated to give crude 2-(4,5-dihydro-7*H*-thieno[2,3-c]pyran-7-yl)-ethanol as a yellowish oil. This was used without further purification in the next reaction. $^1$H-NMR ($CDCl_3$, 200 MHz): δ 1.9-2.3 (2H, m), 2.4-2.7 (2H, m), 2.7-3.0 (1H, m), 3.6-3.9 (1H, m), 3.83 (2H, t, *J* = 5.6 Hz), 4.21 (1H, ddd, *J* = 11.4, 5.8, 1.9 Hz), 4.9-5.1 (1H, m), 6.80 (1H, d, *J* = 5.0 Hz), 7.14 (1H, d, *J* = 5.0 Hz) ppm.

c) *tert*-Butyl [2-(4,5-dihydro-7H-thieno[2,3-c]pyran-7-yl)ethoxy]dimethylsilane

Imidazole (10.67 mmol) and *tert*-butyldimethylsilyl chloride (10.67 mmol) were added sequentially to a solution of 2-(4,5-dihydro-7*H*-thieno[2,3-c]pyran-7-yl)-ethanol (9.70 mmol) dissolved in dry DMF (12 mL). After stirring at room temperature for 2h, water was added and the aqueous phase extracted with diethyl ether (3x). The combined organic phases were dried ($MgSO_4$), filtered and concentrated. The crude product was purified by flash column chromatography

on silica gel ($CH_2Cl_2$/MeOH 2%) to afford *tert*-butyl-[2-(4,5-dihydro-7*H*-thieno[2,3-c]pyran-7-yl)-ethoxy]-dimethylsilane as a yellow oil. $^1$H-NMR ($CDCl_3$, 200 MHz): δ 0.06 (6H, s); 0.89 (9H, s), 1.8-2.2 (2H, m); 2.4-2.7 (1H, m), 2.7-3.0 (1H, m), 3.6-4.0 (3H, m), 4.16 (1H, ddd, *J* = 11.3, 5.6, 2.2 Hz), 4.8-5.0 (1H, m), 6.79 (1H, d, *J* = 5.0 Hz), 7.12 (1H, d, *J* = 5.0 Hz) ppm.

d) 7-[2-(*tert*-Butyldimethylsilanyloxy)ethyl]-4,5-dihydro-7H-thieno[2,3-c]pyran-2-carboxylic acid

*n*-BuLi (4.80 mmol) was added dropwise to a suspension of *tert*-butyl-[2-(4,5-dihydro-7*H*-thieno[2,3-c]pyran-7-yl)ethoxy]dimethylsilane (4.36 mmol) in tetrahydrofuran at -78ºC and the resulting solution stirred for 1h. Gaseous carbon dioxide was bubbled through the solution for 10 min at -78ºC before allowing the reaction to warm to room temperature. The reaction mixture was quenched with saturated aqueous ammonium chloride (caution: exothermic reaction), a mixture of water:brine:diethyl ether (1:1:1) added and extracted with diethyl ether (3x) to afford a yellowish solid. This was triturated with hexane and filtered to give pure 7-[2-(*tert*-butyldimethylsilanyloxy)ethyl]-4,5-dihydro-7*H*-thieno[2,3-c]pyran-2-carboxylic acid as a white solid. $^1$H-NMR ($CDCl_3$, 300 MHz): δ 0.06 (6H, s), 0.89 (9H, s), 1.8-2.2 (2H, m), 2.5-2.7 (1H, m), 2.7-3.0 (1H, m), 3.6-4.0 (3H, m), 4.2 (1H, ddd, *J* = 11.3, 5.6, 2.2 Hz), 4.8-5.0 (1H, m), 7.58 (1H, s) ppm.

e) 7-[2-(*tert*-Butyldimethylsilanyloxy)ethyl]-4,5-dihydro-7*H*-thieno[2,3-c]pyran-2-carboxamide

Carbonyl diimidazole (8.05 mmol) was added to a suspension of 7-[2-(*tert*-butyldimethylsilanyloxy)ethyl]-4,5-dihydro-7*H*-thieno[2,3-c]pyran-2-carboxylic acid (3.22 mmol) in tetrahydrofuran (50 mL) and the resulting solution stirred at room temperature for 24h. The solvent was concentrated *in vacuo* and the residue treated with a solution of ammonia in dioxan (16.0 mmol). The solution was stirred at room temperature for 24h. The solution was evaporated to dryness and the residue partitioned between dichloromethane and water. The aqueous phase was extracted with dichloromethane (3x) and the combined organic layers were dried ($MgSO_4$), filtered and concentrated. The crude product was purified by flash column chromatography on silica gel (ethyl acetate) to afford pure 7-[2-(*tert*-butyldimethylsilanyloxy)ethyl]-4,5-dihydro-7*H*-thieno[2,3-c]pyran-2-carboxamide . $^1$H-NMR ($CDCl_3$, 300 MHz): δ 0.07 (6H, s), 0.90 (9H, s), 1.9-2.1 (2H, m), 2.5-2.7 (1H, m), 2.7-2.9 (1H, m), 3.6-4.0 (3H, m), 4.18 (1H, ddd, *J* = 11.7, 5.8, 2.2 Hz), 4.8-5.0 (1H, m), 6.00 (2H, broad s), 7.27 (1H, s) ppm.

f) 7-(2-Hydroxyethyl)-4,5-dihydro-7*H*-thieno[2,3-c]pyran-2-carboxamide

To a solution of 7-[2-(*tert*-Butyldimethylsilanyloxy)ethyl]-4,5-dihydro-7*H*-thieno[2,3-c]pyran-2-carboxamide (3.64 mmol) in tetrahydrofuran (25 mL) was added tetrabutylammonium fluoride (4 mmol) at 0ºC. The mixture was allowed to warm to room temperature and stirred for 2h. The solution was concentrated *in vacuo* and the crude yellow oil purified by flash column chromatography on silica gel ($CH_2Cl_2$/MeOH 5%) to afford 7-(2-hydroxyethyl)-4,5-dihydro-7*H*-thieno[2,3-c]pyran-2-carboxamide as a white solid. The crude product could also be purified by trituration with a mixture of diethyl ether:hexane (1:1). $^1$H-NMR ($CDCl_3$, 200 MHz): δ 1.8-2.1 (2H, m), 2.51 (1H, dq, *J* = 16.0, 2.0 Hz), 2.7-2.9 (1H, m), 3.6-3.8 (3H, m), 4.14 (1H, ddd, *J* = 11.5, 5.7, 2.2 Hz), 4.89 (1H, dq, *J* = 7.9, 1.9 Hz), 7.26 (1H, s) ppm.

<u>Preparation of 4-(2-hydroxyethyl)-6,7-dihydro-4*H*-thieno[3,2-c]pyran-2-carboxamide</u>

The general procedure for the synthesis of 7-(2-hydroxyethyl)-4,5-dihydro-7H-thieno[2,3-c]pyran-2-carboxamide was followed, using 3-(2-thienyl)ethanol in place of 2-(3-thienyl)ethanol as starting material, and making non-critical variations, to yield the title compound. $^1$H-NMR ($CD_3OD$, 200 MHz): δ 1.7-2.2 (2H, m), 2.7-3.0 (2H, m), 3.6-3.9 (3H, m), 4.17 (1H, ddd, *J* = 11.3, 5.5, 2.8 Hz), 4.7-4.8 (1H, m), 7.42 (1H, s) ppm.

<u>Preparation of methyl 4-(2-hydroxyethyl)-6,7-dihydro-4*H*-thieno[3,2-c]pyran-2-carboxylate</u>

a) Methyl 4-[2-(*tert*-butyldimethylsilanyloxy)ethyl]-6,7-dihydro-4*H*-thieno[3,2-c]pyran-2-carboxylate

n-Butyl lithium (2.87 mmol) was added to a solution of *tert*-butyl-[2-(6,7-dihydro-4*H*-thieno[3,2-c]pyran-4-yl) ethoxydimethylsilane (2.61 mmol) in tetrahydrofuran (15 mL) at -78ºC. The resulting solution was stirred at -78ºC for 1h before adding methyl chloroformate (3.13 mmol). The reaction was allowed to gradually warm to room temperature (aprox. 4h). Saturated aqueous ammonium chloride was added and the aqueous phase extracted with dichloromethane (3x). The combined organic extracts were dried ($MgSO_4$), filtered and evaporated. The residue was purified by flash column chromatography on silica gel (hexane/EtOAc, 95:5) to afford the title carboxylic acid as a colourless oil. $^1$H-NMR ($CDCl_3$, 300 MHz): δ 0.06 (6H, s), 0.90 (9H, s), 1.7-1.9 (1H, m), 2.0-2.2 (1H, m), 2.7-2.8 (1H, m), 2.9-3.1 (1H, m),

3.7-3.8 (2H, m), 3.8-3.9 (4H, m), 4.1-4.2 (1H, m), 4.7-4.8 (1H, m), 7.49 (1H, s) ppm.

b) Methyl 4-(2-hydroxyethyl)-6,7-dihydro-4*H*-thieno[3,2-c]pyran-2-carboxylate

The general procedure for the deprotection of 7-[2-(*tert*-butyldimethylsilanyloxy)ethyl]-[2,3-c]-thienopyran-6-carboxamide was followed, using methyl 4-[2-(*tert*-butyldimethylsilanyloxy)ethyl]-6,7-dihydro-4*H*-thieno[3,2-c]pyran-2-carboxylate as starting material, and making non-critical variations, to yield the title compound. $^1$H-NMR ($CDCl_3$, 200 MHz): δ 1.9-2.0 (1H, m), 2.1-2.2 (1H, m), 2.4-2.5 (1H, m), 2.7-2.8 (1H, m), 3.0-3.2 (1H, m), 3.7-3.9 (6H, m), 4.26 (1H, ddd, *J* = 11.3, 5.6, 1.6 Hz), 4.88 (1H, d, *J* = 8.9 Hz), 7.45 (1H, s) ppm.

Preparation of 7-(2-hydroxyethyl)-7-methyl-4,5-dihydro-7*H*-thieno[2,3-c]pyran-2-carboxamide

a) Ethyl (7-methyl-4,5-dihydro-7*H*-thieno[2,3-c]pyran-7-yl)-acetate

Ethyl acetoacetate (14.33 mmol) and boron trifluoride etherate (6.82 mmol) were added sequentially to a solution of 2-(3-thienyl)ethanol (13.65 mmol) in toluene (8 mL) at 0ºC. The resulting solution was stirred for 2h at 0ºC and an additional 2h at room temperature. Diethyl ether was added and the organic phase washed with water (3x), dried ($MgSO_4$), filtered and evaporated. The crude product was purified by column chromatography on silica gel (hexane/EtOAc 9:1) to afford pure ethyl (7-methyl-4,5-dihydro-7*H*-thieno[2,3-c]pyran-7-yl)-acetate as a colourless oil. $^1$H-NMR ($CDCl_3$, 200 MHz): δ 1.22 (3H, t, *J* = 7.1 Hz), 1.67 (3H, s), 2.6-3.0 (4H, m), 3.9-4.0 (2H, m), 4.13 (2H, c, *J* = 7.1 Hz), 6.75 (1H, d, *J* = 5.1 Hz), 7.13 (1H, d, *J* = 5.1 Hz) ppm.

b) 7-(2-hydroxyethyl)-7-methyl-4,5-dihydro-7*H*-thieno[2,3-c]pyran-2-carboxamide

The general procedure for the synthesis of 7-(2-hydroxy-ethyl)-4,5-dihydro-7*H*-thieno[2,3-c]pyran-2-carboxamide was followed, using ethyl (7-methyl-4,5-dihydro-7*H*-thieno[2,3-c]pyran-7-yl)-acetate as starting material and making non-critical variations, to yield the title compound. $^1$H-NMR ($CD_3OD$, 200 MHz): δ 1.53 (3H, s), 2.09 (2H, t, *J* = 7.6 Hz), 2.6-2.8 (2H, m), 3.4-3.5 (1H, m), 3.6-3.8 (1H, m), 3.8-4.0 (2H, m), 7.37 (1H, s) ppm.

Preparation of 7-(2-Hydroxyethyl)-3-methyl-4,5-dihydro-7*H*-thieno[2,3-c]pyran-2-carboxamide

a) 7-[2-(*tert*-Butyldimethylsilanyloxy)-ethyl]-3-methyl-4,5-dihydro-7H-thieno[2,3-c]pyran-2-carboxylic acid

To a solution of *N, N, N'*, *N'*-tetramethylethylenediamine (3.99 mmol) in tetrahydrofuran (2 mL) was added sec-butyl lithium (3.65 mmol) at -78ºC. After 10min, a solution of 7-[2-(*tert*-butyldimethylsilanyloxy) ethyl] -4,5-dihydro-7*H*-thieno[2,3-c]pyran-2-carboxylic acid (1.14 mmol) in tetrahydrofuran (10 mL) was added and the resulting solution stirred at -78ºC for 90min before addition of iodomethane (3.42 mmol). The reaction mixture was allowed to warm gradually to room temperature (approx. 4 h) before quenching it with saturated aqueous ammonium chloride. The aqueous phase was extracted with dichloromethane (5x) and the combined organic phases dried ($MgSO_4$), filtered and concentrated. The crude product was purified by column chromatography on silica (dichloromethane/methanol 9:1) to obtain pure 7-[2-(*tert*-butyldimethylsilanyloxy)ethyl]-3-methyl-4,5-dihydro-7H-thieno[2,3-c]pyran-2-carboxylic acid as a white solid. $^1$H-NMR ($CDCl_3$, 200 MHz): δ 0.10 (6H, s), 0.92 (9H, s), 1.6-2.0 (2H, m), 2.26 (3H, s), 2.3-2.7 (2H, m), 3.7-4.0 (3H, m), 4.32 (1H, ddd, *J* = 9.5, 6.5, 2.7 Hz), 5.0-5.1 (1H, m) ppm.

b) 7-(2-Hydroxyethyl)-3-methyl-4,5-dihydro-7*H*-thieno[2,3-c]pyran-2-carboxamide

The general procedure for the synthesis of 7-(2-hydroxyethyl)-4,5-dihydro-7*H*-thieno[2,3-*c*]pyran-2-carboxamide was followed, using 7-[2-(*tert*-butyldimethylsilanyloxy)ethyl]-3-methyl-4,5-dihydro-7*H*-thieno[2,3-c]pyran-2-carboxylic acid as starting material and making non-critical variations, to yield the title compound. $^1$H-NMR ($CD_3OD$, 200 MHz): δ 1.8-2.1 (2H, m), 2.33 (3H, s), 2.4-2.5 (1H, m), 2.6-2.8 (1H, m), 3.6-3.9 (3H, m), 4.1-4.3 (1H, m), 4.7-4.8 (1H, m) ppm.

Preparation of 7-(2-hydroxyethyl)-3-chloro-4,5-dihydro-7*H*-thieno[2,3-c]pyran-2-carboxamide

a) 7-[2-(*tert*-Butyldimethylsilanyloxy)-ethyl]-3-chloro-4,5-dihydro-7*H*-thieno[2,3-c]pyran-2-carboxylic acid

Following the procedure used for the synthesis of 7-[2-(*tert*-butyldimethylsilanyloxy)ethyl]-3-methyl-4,5-dihydro-7H-thieno[2,3-c]pyran-2-carboxylic acid and using *N*-chlorosuccinimide instead of iodomethane, the title compound

was obtained as a white solid. $^1H$-NMR ($CDCl_3$, 200 MHz): δ 0.07 (6H, s), 0.89 (9H, s), 1.7-2.1 (2H, m), 2.4-2.9 (2H, m), 3.6-3.95 (3H, m), 4.10-4.30 (1H, m), 4.75-4.95 (1H, m) ppm.

b) 7-[2-(*tert*-Butyldimethylsilanyloxy)ethyl]-3-chloro-4,5-dihydro-7*H*-thieno[2,3-c]pyran-2-carboxamide

To a solution of 7-[2-(*tert*-butyldimethylsilanyloxy)ethyl]-3-chloro-4,5-dihydro-7*H*-thieno[2,3-c]pyran-2-carboxylic acid (1.82 mmol) in THF (6 mL) under inert atmosphere was added triethylamine (3.63mmol) and methanesulphonyl chloride (2.18mmol) at 0º C. After stirring for 30 min., a solution of ammonia in dioxane (0.5M) (9.1mmol) was added and the reaction mixture warmed to room temperature and stirred for 24h. The pH of the reaction mixture was adjusted to approx. 2 by addition of a solution of 1M hydrochloric and the aqueous phase extracted with dichloromethane (3x20mL). The combined organic extracts are dried ($Na_2SO_4$), filtered and evaporated to dryness. The crude mixture was purified by column chromatography on silica gel, eluting with dichloromethane/methanol (9.5:0.5) to afford pure 7-[2-(*tert*-butyldimethylsilanyloxy)ethyl]-3-chloro-4,5-dihydro-7*H*-thieno[2,3-c]pyran-2-carboxamide (19) as a white solid. $^1H$-NMR ($CDCl_3$, 200 MHz): δ 0.07 (6H, s), 0.88 (9H, s), 1.75-2.10 (2H, m), 2.4-2.9 (2H, m), 3.65-4.00 (3H, m), 4.15-4.30 (1H, m), 4.75-4.90 (1H, m), 6.5-6.9 (2H, broad doublet, $CONH_2$) ppm.

c) 7-(2-Hydroxyethyl)-3-chloro-4,5-dihydro-7*H*-thieno[2,3-c]pyran-2-carboxamide

The general procedure for the synthesis of 7-(2-hydroxyethyl)-4,5-dihydro-7H-thieno[2,3-c]pyran-2-carboxamide was followed, using 7-[2-(*tert*-butyldimethylsilanyloxy)ethyl]-3-chloro-4,5-dihydro-7*H*-thieno[2,3-c]pyran-2-carboxylic acid as starting material and making non-critical variations, to yield the title compound. as a white solid. $^1H$-NMR ($CD_3OD$, 200 MHz): δ 1.80-2.15 (2H, m), 2.4-2.8 (2H, m), 3.6-3.9 (3H, m), 4.15-4.30 (1H, m), 4.80-4.95 (1H, m) ppm.

Preparation of 7-(2-hydroxyethyl)-3-fluoro-4,5-dihydro-7*H*-thieno[2,3-c]pyran-2-carboxamide

a) 7-[2-(tert-Butyldimethylsilanyloxy)ethyl]-3-fluoro-4,5-dihydro-7*H*-thieno[2,3-c]pyran-2-carboxylic acid

To a solution of 7-[2-(tert-butyldimethylsilanyloxy)ethyl]-4,5-dihydro-7*H*-thieno[2,3-c]pyran-2-carboxylic acid (2.92mmol) in THF (30mL), cooled to -78ºC under an inert atmosphere, was added *tert*-butyllithium (1.5M in hexanes, 9.34mmol) and stirred for 1h. N-Fluorobenzenesulfonimide (9.34mmol) was then added and the reaction allowed to warm slowly to room temperature. After 2 hours the reaction mixture was acidified to pH 3 by addition of hydrochloric acid (1M) and the aqueous layer extracted with dichloromethane (2x20mL), and the organic extracts dried ($Na_2SO_4$), filtered and evaporated to dryness. The residue was purified by column chromatography on silica gel, eluting with dichloromethane/methanol (9:1) to afford the title compound, contaminated with some starting material (ratio 4/1). This crude product was used in the next step without purification.

b) 7-[2-(*tert*-Butyldimethylsilanyloxy)ethyl]-3-fluoro-4,5-dihydro-7*H*-thieno[2,3-c]pyran-2-carboxamide

The general procedure for the synthesis of 7-[2-(*tert*-butyldimethylsilanyloxy)ethyl]-3-chloro-4,5-dihydro-7*H*-thieno[2,3-c]pyran-2-carboxamide was followed, using 7-[2-(*tert*-butyldimethylsilanyloxy)-ethyl]-3-fluoro-4,5-dihydro-7H-thieno[2,3-c]pyran-2-carboxylic acid acid as starting material and making non-critical variations, to yield the title compound as a white solid, after purification by column chromatography on silica gel, eluing with dichloromethane/methanol (9.5:0.5). $^1H$-NMR ($CDCl_3$, 200 MHz): δ 0.04 (6H, s), 0.86 (9H, s), 1.7-2.1 (2H, m), 2.4-2.8 (2H, m), 3.6-3.9 (3H, m), 4.10-4.25 (1H, m), 4.7-4.8 (1H, m), 6.10-6.35 and 6.7-6.9 (2H, broad doublet, $CONH_2$) ppm.

c) 7-(2-Hydroxyethyl)-3-fluoro-4,5-dihydro-7*H*-thieno[2,3-c]pyran-2-carboxamide

The general procedure for the synthesis of 7-(2-hydroxyethyl)-4,5-dihydro-7*H*-thieno[2,3-c]pyran-2-carboxamide was followed, using 7-[2-(*tert*-butyldimethylsilanyloxy)ethyl]-3-chloro-4,5-dihydro-7*H* thieno[2,3-c]pyran-2-carboxylic acid as starting material and making non-critical variations, to yield the title compound. as a white solid. $^1H$-NMR ($CD_3OD$, 200 MHz): δ 1.80-2.15 (2H, m), 2.4-2.8 (2H, m), 3.6-3.9 (3H, m), 4.15-4.30 (1H, m), 4.80-4.95 (1H, m) ppm.

Preparation of 5,5-difluoro-4-(2-hydroxyethyl)-4,5,6,7-tetrahydrobenzothiophene-2-carboxamide

a) 5,5-Difluoro-4,5,6,7-tetrahydrobenzothiophen-4-one

A solution of 4-keto-4,5,6,7-tetrahydrobenzothiophene (1.18 mmol) in tetrahydrofuran (8 mL) was added to a solution of potassium hexamethyldisilazide in toluene (5.9 mmol) at room temperature. In a separate reaction flask,

N-fluorobenzenesulfonimide (4.70 mmol) dissolved in tetrahydrofuran (15 mL) was added to a suspension of magnesium bromide (4.70 mmol) in tetrahydrofuran (8 mL) and the resulting pinkish suspension cooled to -78ºC. To this solution was added dropwise the benzothiophene anion solution. The resulting dark suspension was stirred at -60ºC for 2h and then allowed to warm gradually to room temperature. Saturated aqueous sodium bicarbonate was added and the aqueous phase extracted with ethyl acetate (4x). The combined organic phases were dried ($MgSO_4$), filtered and concentrated to give pure 5,5-difluoro-4,5,6,7-tetrahydrobenzothiophen-4-one as a yellow oil. $^1$H-NMR ($CDCl_3$, 200 MHz) : δ 2.5-2.8 (2H, ddt, *J* = 14.4, 14.4, 6.1 Hz), 3.27 (2H, t, *J* = 6.1 Hz), 7.20 (1H, d, *J* = 5.4 Hz), 7.44 (1H, d, *J* = 5.4 Hz) ppm.

b) Ethyl (5,5-difluoro-4,5,6,7-tetrahydrobenzothiophen-4-ylidene) acetate

Triethyl phosphonoacetate (3.2 mmol) was added dropwise to a suspension of sodium hydride (3.2 mmol) in dioxane (8 mL). The resulting solution was stirred for 1h at room temperature before adding a solution of 5,5-difluoro-4,5,6,7-tetrahydrobenzothiophen-4-one (1.6 mmol) in tetrahydrofuran (5 mL). The resulting solution was stirred for 1h at room temperature and an additional 12h at 120ºC. Water was added and the aqueous phase extracted with ethyl acetate (3x). The combined organic phases were dried ($MgSO_4$), filtered and concentrated. The crude product was purified by flash column chromatography on silica gel (hexane/ethyl acetate 9:1) to obtain pure ethyl (5,5-difluoro-4,5,6,7-tetrahydrobenzothiophen-4-ylidene) acetate. $^1$H-NMR ($CDCl_3$, 200 MHz): δ 1.33 (3H, t, *J* = 7.1 Hz), 2.44 (2H, ddt, *J* = 13.4, 13.4, 6.5 Hz), 3.14 (2H, t, *J* = 6.5 Hz), 4.27 (2H, c, *J* = 7.1 Hz), 6.29 (1H, t, *J* = 2.0 Hz), 7.13 (1H, d, *J* = 5.4 Hz), 7.61 (1H, d, *J* = 5.4 Hz) ppm.

c) 2-(5,5-Difluoro-4,5,6,7-tetrahydrobenzothiophen-4-ylidene)ethanol

DiBAL-H (1.83 mmol) was added to a solution of ethyl (5,5-difluoro-4,5,6,7-tetrahydrobenzothiophen-4-ylidene)acetate (0.9 mmol) in tetrahydrofuran (10 mL) at 0ºC and the resulting solution left to stir at room temperature for2h. Brine was added and the mixture stirred for 30min at room temperature. The layers were separated and the aqueous phase extracted with ethyl acetate (5x). The combined organic layers were washed with brine, dried ($MgSO_4$), filtered and concentrated. The crude product was purified by column chromatography on silica (hexane/ethyl acetate 7:3) to afford pure 2-(5,5-difluoro-4,5,6,7-dihydrobenzothiophen-4-ylidene)ethanol. $^1$H-NMR ($CDCl_3$, 200 MHz): δ 2.38 (2H, ddt, *J* = 13.3, 13.3, 6.6 Hz), 3.11 (2H, t, *J* = 6.6 Hz), 4.5-4.7 (2H, m), 6.2-6.3 (1H, m), 7.02 (1H, d, *J* = 5.4 Hz), 7.20 (1H, d, *J* = 5.4 Hz) ppm.

d) *tert*-Butyl-[2-(5,5-difluoro-4,5,6,7-tetrahydrobenzothiophen-4-ylidene)ethoxy]dimethylsilane

Imidazole (0.89 mmol) and *tert*-butyldimethylsilyl chloride (0.89 mmol) were added sequentially to a solution of 2-(5,5-difluoro-4,5,6,7-tetrahydrobenzothiophen-4-ylidene)ethanol (0.74 mmol) dissolved in dry DMF (2 mL). After stirring at room temperature overnight, water was added and the aqueous phase extracted with hexane (3x). The combined organic phases were dried ($MgSO_4$), filtered and concentrated. The crude product was purified by flash column chromatography on silica (hexane) to afford tert-butyl-[2-(5,5-difluoro-4,5,6,7-tetrahydrobenzothiophen-4-ylidene)ethoxy]-dimethylsilane. $^1$H-RMN ($CDCl_3$, 200 MHz): δ 0.10 (6H, s), 0.92 (9H, s), 2.37 (2H, ddt, *J* = 13.3, 13.3, 6.6 Hz), 3.10 (2H, t, J = 6.6 Hz), 4.5-4.6 (2H, m), 6.1-6.2 (1H, m), 6.95 (1H, d, *J* = 5.3 Hz), 7.19 (1H, d, J = 5.3 Hz) ppm.

e) 4-[2-(*tert*-Butyldimethylsilanyloxy)-ethylidene]-5,5-difluoro-4,5,6,7-tetrahydrobenzothiophene-2-carboxylic acid

n-BuLi (0.73 mmol) was added dropwise to a solution of *tert*-butyl-[2-(5,5-difluoro-4,5,6,7-tetrahydrobenzothiophen-4-ylidene)ethoxy]dimethylsilane (4.36 mmol) in tetrahydrofuran (6 mL) at -78ºC and the resulting solution stirred for 45min. Gaseous carbon dioxide was bubbled through the solution for 15 min. at -78ºC before allowing the reaction to warm to room temperature. The reaction was quenched with water and the aqueous phase extracted with ethyl acetate (3x). The combined organic phases were dried ($MgSO_4$), filtered and evaporated. The crude product was purified on a short silica gel column (dichloromethane/methanol, 9:1) to afford pure 4-[2-(*tert*-butyldimethylsilanyloxy)-ethylidene]-5,5-difluoro-4,5,6,7-tetrahydrobenzothiophene-2-carboxylic acid as a white solid. $^1$H-RMN ($CDCl_3$, 200 MHz): δ 0.07 (6H, s), 0.89 (9H, s), 2.35 (2H, ddt, *J* = 13.3, 13.3, 6.6 Hz), 3.07 (2H, t, *J* = 6.6 Hz), 4.5-4.6 (2H, m), 6.2-6.4 (1H, m), 7.68 (1H, s) ppm.

f) 4-[2-(*tert*-Butyldimethylsilanyloxy)-ethylidene]-5,5-difluoro-4,5,6,7-tetrahydrobenzothiophene-2-carboxamide

Carbonyl diimidazole (1.40 mmol) was added to a suspension of 4-[2-(*tert*-butyldimethylsilanyloxy)-ethylidene]-5,5-difluoro-4,5,6,7-tetrahydrobenzothiophene-2-carboxylic acid (0.56 mmol) in tetrahydrofuran (7 mL) and the

resulting solution stirred at room temperature for 24h. The solution was concentrated and the residue treated with a solution of ammonia in dioxane (2.8 mmol). The solution was stirred at room temperature for 24h. The dioxane was removed *in vacuo* and the residue partitioned between dichloromethane and water. The aqueous phase was extracted with dichloromethane (3x) and the combined organic layers dried ($MgSO_4$), filtered and evaporated. The crude product was purified on a silica gel column (dichloromethane/methanol, 9:1) to afford pure 4-[2-(*tert*-butyldimethylsilanyloxy) ethylidene]-5,5-difluoro-4,5,6,7-tetrahydrobenzothiophene-2-carboxamide. $^1H$-NMR ($CDCl_3$, 200 MHz): δ 0.08 (6H, s), 0.90 (9H, s), 2.36 (2H, ddt, *J* = 13.2, 13.2, 6.6 Hz), 3.10 (2H, t, *J* = 6.6 Hz), 4.5-4.6 (2H, m), 6.2-6.3 (2H, m), 7.48 (1H, s) ppm.

g) 4-[2-(*tert*-Butyldimethylsilanyloxy)-ethyl]-5,5-difluoro-4,5,6,7-tetrahydrobenzothiophene-2-carboxamide

Palladium on carbon (0.02 mmol) was added to a solution of 4-[2-(*tert*-butyldimethylsilanyloxy)ethylidene]-5,5-difluoro-4,5,6,7-tetrahydrobenzothiophene-2-carboxamide (0.40 mmol) and triethylamine (0.2 mmol) in ethanol (2 mL). Hydrogen was bubbled through the suspension overnight and the reaction filtered through a Celite pad (methanol). The solvent was removed and the residue purified on a silica gel column (hexane/ethyl acetate 8:2) to afford pure 4-[2-(*tert*-butyldimethylsilanyloxy)ethyl]-5,5-difluoro-4,5,6,7-tetrahydrobenzothiophene-2-carboxamide. $^1H$-NMR ($CDCl_3$, 200 MHz): δ 0.06 (6H, s), 0.89 (9H, s), 1.7-2.4 (4H, m), 2.97 (2H, t, *J* = 6.4 Hz), 3.26 (1H, ddt, *J* = 12.7, 12.7, 6.4 Hz), 3.77 (2H, t, J = 6.4 Hz), 6.33 (2H, broad s), 7.35 (1H, s) ppm.

h) 5,5-Difluoro-4-(2-hydroxyethyl)-4,5,6,7-tetrahydrobenzothiophene-2-carboxamide

To a solution of pure 4-[2-(*tert*-butyldimethylsilanyloxy)ethyl]-5,5-difluoro-4,5,6,7-tetrahydrobenzothiophene-2-carboxamide (0.31 mmol) in tetrahydrofuran (3 mL) was added tetrabutylammonium fluoride (0.34 mmol) at 0ºC. The mixture was allowed to warm to room temperature and stirred for 2h. The solution was concentrated and the crude product purified by flash column chromatography on silica gel (dichloromethane/methanol 5%) to afford 5,5-difluoro-4-(2-hydroxyethyl)-4,5,6,7-tetrahydrobenzothiophene-2-carboxamide as a white solid. $^1H$-NMR ($CD_3OD$, 200 MHz): δ 1.7-2.5 (4H, m), 3.09 (2H, t, *J* = 6.7 Hz), 3.3-3.4 (1H, m), 3.7-3.9 (2H, m), 7.58 (1H, s).

Preparation of 4-(7-methoxynaphthalen-1-yl)-1,2,5,6-tetrahydropyridine

a) 4-Bromo-7-methoxy-1,2-dihydronaphthalene

To a methanol suspension (28 mL) of 4A molecular sieves (5.7 g) was added hydrazine hydrate (85%) (114 mmol). After 30min 7-methoxy-1-tetralone (5.7 mmol) dissolved in methanol (28 ml) was added dropwise and the mixture stirred at room temperature for 18h. The reaction was filtered through Celite (diethyl ether), the solvent removed *in vacuo* and the residual hydrazone dried *in vacuo* with heating (30ºC) for immediate use. In another flask, n-butyl lithium (17.1 mmol) was added to a solution of *t*-butanol (17.1 mmol) in 30 mL of tetrahydrofuran at 0ºC. After 5min, copper(II) bromide (34.2 mmol) was added and stirring continued for 20min; the mixture was then diluted with tetrahydrofuran (18 ml) and the crude hydrazone added in portions. The reaction was allowed to warm to room temperature. After 16h an aqueous ammonia solution (3.5%) was added and the organic material extracted with dichloromethane. The residue was purified by silica gel flash chromatography (hexane/ethyl acetate, 95:5) to afford the title compound. $^1H$-NMR (200 MHz, $CDCl_3$):δ 7.15 (d, 1H, *J* = 2.4 Hz), 7.01 (d, 1H, *J* = 8.1 Hz), 6.73 (dd, 1H, *J* = 2.7 and 8.3 Hz), 6.46 (t, 1H, *J* = 4.8 Hz), 3.82 (s, 3H), 2.77 (t, 2H, *J* = 7.8 Hz), 2.40-2.29 (m, 2H) ppm.

b) 1-Bromo-7-methoxy-naphthalene

4-Bromo-7-methoxy-1,2-dihydronaphthalene (1.44 mmol) was dissolved in tetrahydrofuran (12 mL) and 2,3-dichloro-5,6-dicyano-1,4-benzoquinone (2.89 mmol) added. The resulting mixture was stirred at room temperature for 2h. The solvent was removed *in vacuo* and the crude product purified by flash chromatography column on silica gel (hexane ) to afford the title compound. $^1H$-NMR (200 MHz, $CDCl_3$) :δ 7.78-7.71 (m, 3H), 7.52 (d, 1H, *J* = 2.4 Hz), 7.22-7.14 (m, 2H), 3.98 (s, 3H) ppm.

c) 1-Benzyloxycarbonyl-4-(7-methoxynaphthalen-1-yl)-5,6-dihydro-2H-pyridine

To a solution of 1-bromo-7-methoxy-naphthalene (1.16 mmol) in tetrahydrofuran (5 mL) cooled at -78ºC was added n-butyl lithium (1.28 mmol). After 45min, a solution of 1-benzyloxycarbonyl-4-piperidone (1.39 mmol) in tetrahydrofuran (5 mL) was added and the reaction allowed to warm to room temperature. After 30min. water was added and the aqueous layer extracted with dichloromethane and ethyl acetate. The crude product was purified by flash chromatography on silica gel (hexane/ethyl acetate, 85:15 and 80:20) to afford a mixture of the 4-aryl-4-hydroxypiperidine and 4-piperidone. To this mixture dissolved in toluene (10 mL) was added 6N hydrochloric acid (1.6 mL).and the resulting solution heated at 130ºC for 20h. Water was added and the organic layer separated and washed with saturated aqueous sodium bicarbonate. The combined aqueous layers were basified with saturated sodium bicarbonate and extracted with dichloromethane and ethyl acetate. The organic phase was dried ($MgSO_4$), filtered and concentrated. The crude product was purified by column chromatography on silica (hexane/ethyl acetate, 80:20) to afford the title compound. $^1$H-NMR (200MHz, $CDCl_3$) :δ7.76(d, 1H, *J* = 8.6 Hz), 7.69 (dd, 1H, *J* = 1.6 and 7.8 Hz), 7.43-7.12 (m, 4H), 5.79 (bs, 1H), 5.23 (s, 2H), 4.23 (q, 2H, J = 2.7 Hz), 3.88 (s, 3H), 3.81 (t, 2H, *J* = 5.6 Hz), 2.55 (bs, 2H) ppm.

d) 4-(7-Methoxynaphthalen-1-yl)-1,2,5,6-tetrahydropyridine

To a suspension of 5% palladium on carbon (0.03 mmol) in ethanol (3 mL) was added 1-benzyloxycarbonyl-4-(7-methoxynaphthalen-1-yl)-5,6-dihydro-2*H*-pyridine (0.56 mmol). The reaction was stirred under a hydrogen atmosphere at room temperature for 24h. The reaction mixture was filtered through Celite (methanol) and the solvent evaporated to afford piperidine 4-(7-methoxynaphthalen-1-yl)-1,2,5,6-tetrahydropyridine, which was used without further purification. $^1$H-NMR ($CDCl_3$, 200 MHz): δ 7.76 (d, 1H, *J* = 8.8 Hz), 7.75-7.71 (m, 1H), 7.34-7.23 (m, 3H), 7.16 (dd, 1H, *J* = 2.4 and 8.9 Hz), 5.84 (bs, 1H), 3.98 (s, 3H), 3.96 (m, 3H), 3.53 (t, 2H, *J* = 6.4 Hz), 2.91-2.88 (m, 2H).

General procedure for preparation of substituted 1-(naphthalen-1-yl)-3-methylpiperazines

a) Substituted 3,4-dihydronaphthalen-1-yl trifluoromethane sulfonate

To a solution of the tetralone (1 eq) in tetrahydrofuran (1.2 mL/mmol tetralone) cooled to -78ºC was added lithium hexamethyldisilazide (1.2 eq) in tetrahydrofuran (6 mL/mmol tetralone), and the resulting solution stirred for 1h. N-Phenyl triflimide (1.2 eq) was added in one portion was added to this solution and the reaction allowed to warm to room temperature. Stirring was continued for 2h and after solvent removal, the residue was dissolved in ethyl acetate and washed with 2N aqueous sodium hydroxide. The organic phase was dried ($MgSO_4$), filtered and evaporated. The crude product was purified by column chromatography on silica (hexane) to afford the required product. This procedure was used for the preparation of:

6-Fluoro-3,4-dihydronaphthalen-1-yl trifluoromethanesulfonate
7-Methoxy-3,4-dihydronaphthalen-1-yl trifluoromethanesulfonate

b) Substituted naphthalen-1-yl trifluoromethane sulfonate

2,3-Dichloro-5,6-dicyano-1,4-benzoquinone (1.5 eq) was added to a solution of the substituted 3,4-dihydronaphtalen-1-yl trifluoromethanesulfonate (1 eq) in dioxane (7 mL/mmol triflate) and the resulting suspension heated at reflux for 2 days. The solution was concentrated to low volume and the residue purified by column chromatography on silica (hexane) to afford the required product.
This procedure was used for the preparation of: 6-Fluoronaphthalen-1-yl trifluoromethanesulfonate 7-Methoxynaphthalen-1-yl trifluoromethanesulfonate
Alternatively for 7-fluoronaphthalen-1-yl trifluoromethane sulfonate, the following procedure was used:

Potassium t-butoxide (11.75 mmol) was added in one portion to a solution of 7-fluoru-1-naphthol (10.68 mmol) in tetrahydrofuran (60 mL) at 0ºC. The cooling bath was removed and the yellow solution stirred for 15min at room temperature. N-phenyl triflimide (11.75 mmol) was added in one portion to this solution and the resulting orange mixture stirred for 1h at room temperature. The reaction was quenched by addition of saturated aqueous potassium carbonate and the resulting suspension stirred for 30 minutes before adding hexane. The organic phase was washed with saturated aqueous potassium carbonate (5x), dried ($MgSO_4$), filtered and concentrated. The crude product was purified by flash column chromatography on silica gel (hexane) to give 7-fluoro-naphthalen-1-yl trifluoromethanesulfonate.

c) Substituted 1-(naphthalen-1-yl)-3-methylpiperazines

2-Methylpiperazine (1.2 eq), (R)-BINAP (7.5%), palladium(II)acetate (5%) and cesium carbonate (1.4 eq) were added to a solution of the appropriate trifluoromethanesulfonate (1 eq) in toluene (0.5M). The resulting suspension was heated at 110ºC for 16h. Upon cooling, the mixture was filtered through a short Celite pad (ethyl acetate), the filtrate concentrated and the crude product purified by flash column chromatography on silica (dichloromethane/methanol, 7:3) to afford the required 1-(naphthalen-1-yl)-3-methylpiperazine.

This procedure was used for the preparation of:

1-(6-Fluoro-naphthalen-1-yl)-3-(R)-methylpiperazine
$^1$H-NMR ($CDCl_3$, 200 MHz): δ 1.13 (3H, d, *J* = 6.2 Hz), 2.48 (1H, t, *J* = 11.1 Hz), 2.7-2.9 (2H, m), 3.1-3.3 (5H, m), 6.99 (1H, dd, *J* = 6.9, 1.4 Hz), 7.22 (1H, td, *J* = 8.9, 2.6 Hz), 7.3-7.5 (3H, m), 8.18 (1H, dd, *J* = 9.2, 5.8 Hz) ppm.
1-(6-Fluoro-naphthalen-1-yl)-3-(S)-methylpiperazine
$^1$H-NMR ($CDCl_3$, 200 MHz): δ 1.10 (3H, d, *J* = 6.3 Hz), 1.96 (1H, broad s), 2.44 (1H, t, *J* = 10.8 Hz), 2.77 (1H, td, *J* = 11.6, 2.8 Hz), 3.0-3.2 (5H, m), 6.99 (1H, dd, *J* = 6.8, 1.6 Hz), 7.22 (1H, ddd, *J* = 9.2, 8.4, 2.6 Hz), 7.3-7.5 (3H, m), 8.20 (1H, dd, *J* = 9.3, 5.8 Hz) ppm.
1-(6-Fluoro-naphthalen-1-yl)-3,3-dimethylpiperazine
$^1$H-NMR ($CDCl_3$, 200 MHz): δ 1.61 (6H, broad s), 3.05 (2H, s), 3.25 (2H, broad s), 3.47 (2H, broad s), 7.08 (1H, d, *J* = 6.7 Hz), 7.27 (1H, ddd, *J* = 9.2, 8.3, 2.6 Hz), 7.4-7.5 (2H, m), 7.56 (1H, d, *J* = 8.2 Hz), 8.15 (1H, dd, *J* = 9.3, 5.7 Hz) ppm.
1-(7-Fluoro-naphthalen-1-yl)-3-(R)-methylpiperazine
$^1$H-NMR ($CDCl_3$, 200 MHz): δ 1.15 (3H, d, *J* = 6.2 Hz), 2.4-2.6 (2H, m), 2.8-2.9 (1H, m), 3.1-3.3 (5H, m), 7.11 (1H, d, *J* = 7.4 Hz), 7.23 (1H, td, *J* = 8.5, 2.7 Hz), 7.35 (1H, t, *J* = 7.8 Hz), 7.54 (1H, d, *J* = 8.2 Hz), 7.7-7.9 (2H, m) ppm.
1-(7-Fluoro-naphthalen-1-yl)-3-(S)-methyl-piperazine
$^1$H-NMR ($CDCl_3$, 200 MHz): δ 1.21 (3H, d, *J* = 6.3 Hz), 2.5-2.6 (1H, m), 2.8-3.0 (1H, m), 3.2-3.5 (7H, m), 7.14 (1H, d, *J* = 7.4 Hz), 7.24 (1H, td, *J* = 8.6, 2.7 Hz), 7.37 (1H, t, *J* = 7.8 Hz), 7.56 (1H, d, *J* = 8.2 Hz), 7.7-7.9 (2H, m) ppm.

When the alkyl substituent on the piperazine is other than methyl, the appropriate 2-alkyl piperazine starting material may be prepared according to the procedure of Mickelson *et al*., J. Org. Chem., 1995, 60, 4177-4183. Thus 2-ethylpiperazine was reacted with 6-fluoro-naphthalene-1-yl trifluoromethanesulfonate, as described above, to yield 1-(6-fluoro-naphthalene-1-yl)-3-ethylpiperazine.

<u>General procedure for condensation of alcohols with aryl piperidines or piperazines</u>

To a solution of the alcohol (1 eq) in DMF (0.3M) was added methanesulfonyl chloride (1.05 eq) at 0ºC and the resulting yellowish solution stirred at room temperature for 1h. The DMF was removed *in vacuo* and the residue redissolved in acetonitrile (0.2M). Anhydrous potassium carbonate (2 eq) and the piperidine or piperazine (1.1 eq) were added to this solution and the resulting suspension heated at 80ºC for 24h. Water was added and the aqueous phase extracted with dichloromethane (3x). The combined organic phases were dried ($MgSO_4$), filtered. and concentrated. The crude product was purified on a silica gel column (dichloromethane/methanol 95:5) to afford the pure coupled product.

Using this procedure the following examples were prepared:

<u>Example 1</u> 4-{2-[4-(6-Fluoro-1*H*-indol-3-yl)-3,6-dihydro-2*H*-pyridin-1-yl]ethyl}-6,7-dihydro-4*H*-thieno[3,2-c]pyran-2-carboxamide
$^1$H-NMR (DMSO-$d_6$, 500 MHz): δ 1.8-1.9 (1H, m), 2.0-2.1 (1H, m), 2.6-2.7 (4H, m), 2.8-2.9 (1H, m), 3.1-3.2 (2H, m), 3.66 (1H, td, *J* = 10.4, 3.1 Hz), 4.10 (1H, ddd, *J* = 11.1, 5.4, 2.6 Hz), 4.66 (1H, d, *J* = 7.0 Hz), 6.09 (1H, s), 6.86 (1H, td, *J* = 9.2, 2.3 Hz), 7.12 (1H, dd, *J* = 10.1, 2.3 Hz), 7.28 (1H, broad s), 7.36 (1H, d, *J* = 2.6 Hz), 7.57 (1H, s), 7.7-7.8 (2H, m), 11.15 (1H, s) ppm.

<u>Example 2</u> Methyl 4-{2-[4-(6-fluoro-1*H*-indol-3-yl)-3,6-dihydro-2*H*-pyridin-1-yl]ethyl}-6,7-dihydro-4*H*-thieno[3,2-c] pyran-2-carboxylate
$^1$H-NMR ($CDCl_3$, 500 MHz): δ 1.9-2.0 (1H, m), 2.1-2.2 (1H, m), 2.5-2.8 (7H, m), 2.9-3.0 (1H, m), 3.1-3.3 (2H, m), 3.76 (1H, td, *J* = 10.5, 3.7 Hz), 3.84 (3H, s), 4.20 (1H, ddd, *J* = 11.4, 5.5, 2.7 Hz), 4.74 (1H, d, *J* = 6.5 Hz), 6.13 (1H, s), 6.87 (1H, td, *J* = 9.2, 2.3 Hz), 7.02 (1H, dd, *J* = 9.4, 2.3 Hz), 7.10 (1H, d, *J* = 2.3 Hz), 7.49 (1H, s), 7.76 (1H, dd, *J* = 8.9, 5.3 Hz), 8.25 (1H, broad s) ppm.

<u>Example 3</u> 7-{2-[4-(6-Fluoro-1*H*-indol-3-yl)-3,6-dihydro-2*H*-pyridin-1-yl] ethyl}-4,5-dihydro-7*H*-thieno [2,3-c]pyran-2-carboxamide
$^1$H-NMR (DMSO-$d_6$, 200 MHz): δ 1.8-2.1 (2H, m), 2.5-2.8 (8H, m), 3.12 (2H, broad s), 3.6-3.8 (1H, m), 4.1-4.2 (1H, m), 4.7-4.9 (1H, m), 6.10 (1H, broad s), 6.86 (1H, td, *J* = 9.3, 2.2 Hz), 7.12 (1H, dd, *J* = 10.0, 2.3 Hz), 7.36 (1H, d, *J* = 2.3 Hz), 7.48 (1H, s), 7.77 (1H, dd, *J* = 8.8, 5.4 Hz), 7.87 (1H, broad s), 11.15 (1H, broad s) ppm.

<u>Example 4</u> 7-{2-[2-(S)-Methyl-4-(naphthalen-1-yl)piperazin-1-yl]ethyl}-4,5-dihydro-7*H*-thieno[2,3-c]pyran-2-car-

boxamide
$^1H$-NMR ($CDCl_3$, 200 MHz): δ 1.18 (3H, d, *J* = 6.0 Hz), 2.0-2.1 (2H, m), 2.5-3.2 (11H, m) , 3.76 (1H, td, *J* = 10.8, 3.9 Hz), 4.21 (1H, dd, *J* = 9.3, 5.5 Hz), 4.8-4.9 (1H, m), 5.74 (2H, broad s), 7.07 (1H, dd, *J* = 7.3, 0.9 Hz), 7.25 (1H, s), 7.3-7.6 (4H, m), 7.7-7.9 (1H, m), 8.1-8.3 (1H, m) ppm.

Example 5 7-{2-[2-(R)-Methyl-4-(naphthalen-1-yl)piperazin-1-yl]ethyl}-4,5-dihydro-7*H*-thieno[2,3-c]pyran-2-carboxamide.
$^1H$-NMR ($CDCl_3$, 200 MHz): δ 2.19 (3H, d, *J* = 6.0 Hz), 2.0-2.1 (2H, m), 2.5-3.2 (11H, m), 3.76 (1H, td, *J*= 10.5, 3.9 Hz), 4.1-4.3 (1H, m), 4.8-4.9 (1H, m), 5.96 (2H, broad s), 7.07 (1H, dd, *J* = 7.3, 0.8 Hz), 7.26 (1H, s), 7.3-7.6 (4H, m), 7.7-7.9 (1H, m), 8.1-8.3 (1H, m) ppm.

Example 6 7-{2-[4-(6-Fluorobenzothiophen-3-yl)-2-(R)-methylpiperazin-1-yl]ethyl}-4,5-dihydro-7*H*-thieno[2,3-c]pyran-2-carboxamide.
$^1H$-NMR ($CDCl_3$, 200 MHz): δ 1.15 (3H, d, *J* = 5.2 Hz), 1.9-2.1 (2H, m), 2.4-3.3 (11H, m), 3.72 (1H, td, *J* = 10.8, 3.7 Hz), 4.18 (1H, dd, *J* = 11.2, 4.0 Hz), 4.8-4.9 (1H, m), 6.32 (2H, broad s), 6.51 (1H, s), 7.08 (1H, td, *J* = 8.8, 2.3 Hz), 7.26 (1H, s), 7.43 (1H, dd, J = 8.8, 2.3 Hz), 7.65 (1H, dd, *J* = 8.8, 5.2 Hz) ppm.

Example 7 7-{2-[4-(6-Fluorobenzothiophen-3-yl)-2-(S)-methylpiperazin-1-yl]ethyl}-4,5-dihydro-7*H*-thieno[2,3-c]pyran-2-carboxamide.
$^1H$-NMR ($CDCl_3$, 200 MHz) : δ 1.17 (3H, d, *J* = 6.0 Hz), 2.0-2.1 (2H, m), 2.5-3.1 (9H, m), 3.2-3.4 (2H, m), 3.75 (1H, td, *J* = 11.0, 4.0 Hz), 4.1-4.3 (1H, m), 4.8-4.9 (1H, m), 5.83 (2H, broad s), 6.54 (1H, s), 7.09 (1H, td, *J* = 8.8, 2.3 Hz), 7.26 (1H, s), 7.45 (1H, dd, *J* = 8.8, 2.3 Hz), 7.67 (1H, dd, *J* = 8.8, 5.2 Hz) ppm.

Example 8 7-{2-[4-(6-Fluoronaphthalen-1-yl)-2-(R)-methylpiperazin-1-yl]ethyl}-4,5-dihydro-7*H*-thieno[2,3-c]pyran-2-carboxamide.
$^1H$-NMR ($CDCl_3$, 200 MHz): δ 1.17 (3H, d, *J* = 5.9 Hz), 1.9-2.1 (2H, m), 2.5-3.2 (11H, m), 3.75 (1H, td, *J* = 10.6, 3.9 Hz), 4.1-4.3 (1H, m), 4.8-4.9 (1H, m), 6.14 (2H, broad s), 7.02 (1H, dd, *J* = 6.8, 1.3 Hz), 7.22 (1H, td, *J* = 8.9, 2.5 Hz), 7.26 (1H, s), 7.3-7.5 (3H, m), 8.21 (1H, dd, *J* = 9.2, 5.8 Hz) ppm.

Example 9 7-{2-[4-(6-Fluoronaphthalen-1-yl)-2-(S)-methylpiperazin-1-yl]ethyl}-4,5-dihydro-7*H*-thieno[2,3-c]pyran-2-carboxamide.
$^1H$-NMR ($CDCl_3$, 200 MHz): δ 1.17 (3H, d, *J* = 5.9 Hz), 2.0-2.1 (2H, m), 2.5-3.2 (11H, m), 3.75 (1H, td, *J* = 10.6, 3.8 Hz), 4.2-4.3 (1H, m), 4.8-4.9 (1H, m), 6.19 (2H, broad s), 7.01 (1H, dd, *J* = 6.9, 1.4 Hz), 7.22 (1H, td, *J* = 9.0, 2.5 Hz), 7.26 (1H, s), 7.3-7.5 (3H, m), 8.21 (1H, dd, J = 9.2, 5.8 Hz) ppm.

Example 10 7-{2-[4-(7-Fluoronaphthalen-1-yl)-2-(R)-methylpiperazin-1-yl]ethyl}-4,5-dihydro-7*H*-thieno[2,3-c]pyran-2-carboxamide.
$^1H$-NMR ($CDCl_3$, 200 MHz): δ 1.18 (3H, d, *J* = 5.3 Hz), 2.0-2.2 (2H, m), 2.5-3.2 (11H, m), 3.76 (1H, td, *J* = 10.8, 3.9 Hz), 4.21 (1H, dd, *J* = 11.1, 3.6 Hz), 4.8-4.9 (1H, m), 5.98 (2H, broad *s*), 7.11 (1H, d, *J* = 7.3 Hz), 7.22 (1H, td, *J* = 8.7, 2.5 Hz), 7.27 (1H, s), 7.35 (1H, t, *J* = 7.5 Hz), 7.53 (1H, d, *J* - 8.2 Hz) ppm.

Example 11 7-{2-[4-(7-Fluoro-naphthalen-1-yl)-2-(S)-methyl-piperazin-1-yl]ethyl}-4,5-dihydro-7*H*-thieno[2,3-c]pyran-2-carboxamide.
$^1H$-NMR ($CDCl_3$, 200 MHz): δ 1.18 (3H, d, *J* = 4.5 Hz), 2.0-2.1 (2H, m), 2.5-3.2 (11H, m), 3.76 (1H, td, *J* = 10.5, 4.0 Hz), 4.22 (1H, dd, *J* = 11.5, 4.3 Hz), 4.8-4.9 (1H, m), 5.96 (2H, broad s), 7.11 (1H, d, *J* = 7.4 Hz), 7.21 (1H, td, *J* = 8.8, 2.6 Hz), 7.27 (1H, s), 7.35 (1H, t, *J* = 7.8 Hz), 7.53 (1H, d, *J* = 8.2 Hz), 7.7-7.9 (2H, m) ppm.

Example 12 7-{2-[4-(4-Fluoronaphthalen-1-yl)-2-(R)-methylpiperazin-1-yl]ethyl}-4,5-dihydro-7*H*-thieno[2,3-c]pyran-2-carboxamide.
$^1H$-NMR ($CDCl_3$, 200 MHz): δ 1.17 (3H, d, *J* = 4.5 Hz), 2.0-2.2 (2H, m), 2.5-3.2 (11H, m), 3.76 (1H, td, *J* = 11.0, 3.9 Hz), 4.1-4.3 (1H, m), 4.8-4.9 (1H, m), 5.92 (2H, broad s), 6.9-7.1 (2H, m), 7.26 (1H, s), 7.5-7.6 (2H, m), 8.0-8.1 (1H, m), 8.2-8.3 (1H, m) ppm.

Example 13 7-{2-[4-(4-Fluoronaphthalen-1-yl)-2-(S)-methylpiperazin-1-yl]ethyl}-4,5-dihydro-7*H*-thieno[2,3-c]pyran-2-carboxamide.
$^1H$-NMR ($CDCl_3$, 200 MHz): δ 1.17 (3H, d, *J* = 5.9 Hz), 2. 0-2.2 (2H, m), 2.5-3.2 (11H, m), 3.76 (1H, td, *J* = 11.0, 4.0 Hz), 4.1-4.3 (1H, m), 4.8-4.9 (1H, m), 5.98 (2H, broad s), 6.9-7.1 (2H, m), 7.26 (1H, s), 7.4-7.6 (2H, m), 8.0-8.1 (1H, m), 8.2-8.3 (1H, m) ppm.

Example 14 4-{2-[4-(6-Fluoronaphthalen-1-yl)-2-(R)-methylpiperazin-1-yl]ethyl}-6,7-dihydro-4H-thieno[3,2-c]pyran-2-carboxamide.
$^1$H-NMR ($CDCl_3$, 200 MHz): δ 1.16 (3H, d, *J* = 5.0 Hz), 1.9-2.2 (2H, m), 2.5-3.2 (11H, m), 3.77 (1H, ddd, *J* = 11.4, 9.6, 3.9 Hz), 4.24 (1H, ddd, *J* = 11.0, 5.5, 2.6 Hz), 4.6-4.8 (1H, m), 7.03 (1H, d, *J* = 6.9 Hz), 7.1-7.5 (5H, m), 8.21 (1H, dd, *J* = 9.2, 5.9 Hz) ppm.

Example 15 4-{2-(4-(6-Fluoronaphthalen-1-yl)-2-(S)-methylpiperazin-1-yl]ethyl}-6,7-dihydro-4*H*-thieno[3,2-c]pyran-2-carboxamide.
$^1$H-NMR ($CDCl_3$, 200 MHz): δ 1.17 (3H, d, *J* = 5.6 Hz), 1.8-2.2 (2H, m), 2.5-3.2 (11H, m), 3.78 (1H, ddd, *J* = 11.4, 9.6, 3.9 Hz), 4.23 (1H, ddd, *J* = 11.3, 5.5, 2.8 Hz), 4.7-4.8 (1H, m), 5.72 (2H, broad s), 7.03 (1H, dd, *J* = 6.9, 1.5 Hz), 7.23 (1H, ddd, *J* = 9.2, 8.3, 2.6 Hz), 7.31 (1H, d, *J* = 6.0 Hz), 7.3-7.5 (3H, m), 8.21 (1H, dd, *J* = 9.2, 5.8 Hz) ppm.

Example 16 7-{2-[4-(6-Fluoronaphthalen-1-yl)-2-(R)-methylpiperazin-1-yl]ethyl}-7-methyl-4,5-dihydro-7*H*-thieno[2,3-c]pyran-2-carboxamide.
$^1$H-NMR ($CDCl_3$, 200 MHz): δ 1.10 (3H, d, *J* = 5.8 Hz), 1.56 (3H, s), 2.0-2.1 (2H, m), 2.3-2.4 (1H, m), 2.6-3.2 (10H, m), 3.9-4.0 (2H, m), 5.73 (2H, broad s), 7.99 (1H, dd, *J* = 6.8, 1.4 Hz), 7.1-7.2 (2H,m), 7.3-7.5 (3H, m), 8.16 (1H, dd, *J* = 8.8, 5.7 Hz) ppm.

Example 17 7-{2 -[4-(6-Fluoronaphthalen-1-yl)-2-(S)-methylpiperazin-1-yl]ethyl}-7-methyl-4,5-dihydro-7*H*-thieno[2,3-c]pyran-2-carboxamide.
$^1$H-NMR ($CDCl_3$, 200 MHz): δ 1.11 (3H, d, *J* = 5.8 Hz), 1.5-1.6 (3H, m), 2.0-2.1 (2H, m), 2.3-2.5 (1H, m), 2.5-3.3 (10H, m), 3.8-4.1 (2H, m), 5.73 (2H, broad s), 7.01 (1H, dd, J = 6.8, 1.4 Hz), 7.2-7.3 (2H, m), 7.3-7.5 (3H, m), 8.19 (1H, dd, J = 8.8, 5.7 Hz) ppm.

Example 18 7-{2-[4-(6-Fluoronaphthen-1-yl)-2-(R)-methylpiperazin-1-yl]ethyl}-3-methyl-4,5-dihydro-7*H*-thieno[2,3-c]pyran-2-carboxamide.
$^1$H-NMR ($CDCl_3$, 200 MHz): δ 1.18 (3H, d, *J* = 7.1 Hz), 1.9-2.1 (2H, m), 2.4 (3H, s), 2.4-2.9 (5H, m), 3.0-3.2 (5H, m), 3.78 (1H, d, *J* = 11.0, 4.1 Hz), 4.2-4.3 (1H, m), 4.8-4.9 (1H, m), 5.59 (2H, broad s), 7.02 (1H, dd, *J* = 7.0, 1.5 Hz), 7.2-7.3 (1H, m), 7.3-7.5 (3H, m), 8.21 (1H, dd, *J* = 9.1, 6.0 Hz) ppm.

Example 19 7-{2-[4-(6-Fluoronaphthen-1-yl)-2-(R)-methylpiperazin-1-yl]ethyl}-3-chloro-4,5-dihydro-7*H*-thieno[2,3-c]pyran-2-carboxamide.
Isomer 1: $^1$H-NMR ($CDCl_3$, 200 MHz): δ 1.22 (3H, d, *J* = 6.2 Hz), 1.9-2.2 (2H; m), 2.45-3.30 (11H, m), 3.79 (1H, td, *J* = 10.9 and 4.1 Hz), 4.20-4.35 (1H, m), 4.75-4.90 (1H, m), 7.03 (1H, dd, *J* = 6.9 and 1.5 Hz), 7.15-7.30 (1H, m) , 7.3-7.5 (3H, m), 8.21 (1H, dd, *J* = 9.3 and 5.8 Hz) ppm.
Isomer 2: $^1$H-NMR ($CDCl_3$, 200 MHz): δ 1.21 (3H, d, *J* = 6.2 Hz), 1.9-2.2 (2H, m), 2.45-3.30 (11H, m), 3.78 (1H, td, *J* = 10.2 and 4.3 Hz), 4.20-4.35 (1H, m), 4.70-4.85 (1H, m), 7.02 (1H, dd, *J* = 7 and 1.35 Hz), 7.15-7.30 (1H, m), 7.3-7.5 (3H, m), 8.21 (1H, dd, J = 9.2 and 5.8 Hz) ppm.

Example 20 7-{2-[4-(6-Fluoronaphthen-1-yl)-2-(R)-methylpiperazin-1-yl]ethyl}-3-fluoro-4,5-dihydro-7*H*-thieno[2,3-c]pyran-2-carboxamide.
Isomer 1: $^1$H-NMR ($CDCl_3$, 200 MHz): δ 1.17 (3H, d, *J* = 5.9 Hz), 1.9-2.2 (2H, m), 2.45-3.30 (11H, m), 3.76 (1H, td, *J* = 11 and 4.1 Hz), 4.20-4.35 (1H, m), 4.75-4.85 (1H, m), 5.5-6.4 (2H, broad d), 7.03 (1H, dd, *J* = 6.85 and 1.5 Hz), 7.15-7.30 (1H, m), 7.35-7.55 (3H, m), 8.21 (1H, dd, J = 9.3 and 5.8 Hz) ppm.
Isomer 2: $^1$H-NMR ($CDCl_3$, 200 MHz): δ 1.18 (3H, d, *J* = 5.4 Hz), 1.85-2.20 (2H, m), 2.45-3.30 (11H, m), 3.76 (1H, td, *J* = 10.8 and 4.1 Hz), 4.2-4.3 (1H, m), 4.7-4.8 (1H, m), 5.5-6.4 (2H, broad d), 7.03 (1H, d, *J* = 7 Hz), 7.15-7.30 (1H, m), 7.3-7.5 (3H, m), 8.21 (1H, dd, *J* = 9.1 and 5.7 Hz) ppm.

Example 21 5,5-Difluoro-4-{2-[4-(6-fluoro-naphthalen-1-yl)-2-methyl-piperazin-1-yl]ethyl}-4,5,6,7-tetrahydro benzothiophene-2-carboxamide.
$^1$H-NMR ($CDCl_3$, 200 MHz): δ 1.19 (3H, d, *J* = 5.8 Hz), 1.6-3.4 (16H, m), 5.69 (2H, broad s), 7.2-7.3 (1H, m), 7.3-7.5 (4H, m), 8.21 (1H, dd, *J* = 9.3, 5.7 Hz) ppm.

Example 22 7-{2-[4-(7-Methoxynaphthalen-1-yl)-2-(R)-methylpiperazin-1-yl]ethyl}-4,5-dihydro-7*H*-thieno[2,3-c]pyran-2-carboxamide.
$^1$H-NMR ($CDCl_3$, 200 MHz): δ 1.2-1.3 (3H, m), 2.1-2.2 (2H, m), 2.6-3.3 (11H, m), 3.7-3.8 (1H, m), 3.95 (3H, s), 4.2-4.3 (1H, m), 4.9-5.0 (1H, m), 5.85 (2H, broad s), 7.1-7.2 (2H, m), 7.2-7.3 (2H, m), 7.5-7.6 (2H, m), 7.75 (1H, d, J = 9.0 Hz) ppm.

Example 23 7-{2-[4-(7-Methoxynaphthalen-1-yl)-2-(S)-methylpiperazin-1-yl]ethyl}-4,5-dihydro-7*H*-thieno[2,3-c]pyran-2-carboxamide.
$^1$H-NMR ($CDCl_3$, 200 MHz): δ 1.2-1.3 (3H, m), 2.1-2.2 (2H, m), 2.6-3.3 (11H, m), 3.8-3.9 (1H, m), 3.98 (3H, s), 4.2-4.3 (1H, m), 4.9-5.0 (1H, m), 5.82 (2H, broad s), 7.1-7.2 (2H, m), 7.2-7.3 (2H, m), 7.5-7.6 (2H, m), 7.77 (1H, d, J = 9.0 Hz) ppm.

Example 24 7-{2-[4-(7-Methoxynaphthalen-1-yl)-5,6-dihydropyridin-1-yl]ethyl}-4,5-dihydro-7*H*-thieno[2,3-c]pyran-2-carboxamide.
$^1$H-NMR ($CDCl_3$, 200 MHz): δ 2.1-2.3 (2H, m), 2.5-2.7 (3H, m), 2.7-3.0 (5H, m), 3.2-3.3 (2H, m), 3.77 (1H, ddd, *J* = 11.3, 10.2, 4.0 Hz), 3.89 (3H, s), 4.21 (1H, ddd, *J* = 11.4, 5.6, 2.4 Hz), 4.89 (1H, m), 5.78 (1H, m), 5.92 (2H, broad s), 7.14 (1H, dd, *J* = 8.9, 2.6 Hz), 7.2-7.3 (4H, m), 7.6-7.7 (1H, m), 7.74 (1H, d, *J* = 9.0 Hz) ppm.

Example 25 4-{2-[4-(6-Fluoronaphthalen-1-yl)-2-(R)-ethylpiperazin-1-yl]ethyl}-6,7-dihydro-4*H*-thieno[3,2-c]pyran-2-carboxamide.
$^1$H-NMR ($CDCl_3$, 300 MHz): δ 0.93 (3H, t, *J* = 7.5 Hz), 1.40-1.85 (2H, m), 1.90-2.05 (1H, m), 2.05-2.20 (1H, m), 2.55-2.9 (5H, m), 3.0-3.3 (6H, m), 3.79 (1H, ddd, *J* = 3.9, 10.0, 11.4 Hz), 4.24 (1H, ddd, *J* = 2.6, 5.6, 11.4 Hz), 4.76 (1H, dd, *J* = 2.8, 6.4 Hz), 5.58 (2H, broad s), 7.01 (1H, d, *J* = 7.2 Hz), 7.20-7.30 (2H, m), 7.30-7.49 (3H, m), 8.20 (1H, dd, *J* = 5.8, 9.3 Hz) ppm.

The following Examples illustrate typical formulations containing the compound of the invention.

EXAMPLE 26

Tablets each containing 10 mg of active ingredient are made up as follows:

| | |
|---|---|
| Active ingredient | 10 mg |
| Starch | 160 mg |
| Microcrystalline cellulose | 100 mg |
| Polyvinylpyrrolidone (as 10% solution in water) | 13 mg |
| Sodium carboxymethyl starch | 14 mg |
| Magnesium stearate | 3 mg |
| Total | 300 mg |

The active ingredient, starch and cellulose are mixed thoroughly. The solution of polyvinylpyrrolidone is mixed with the resultant powders and passed through a sieve. The granules so produced are dried and re-passed through a sieve. The sodium carboxymethyl starch and magnesium stearate are then added to the granules which, after mixing, are compressed on a tablet machine to yield tablets each weighing 300 mg.

EXAMPLE 27

Capsules each containing 20 mg of medicament are made as follows:

| | |
|---|---|
| Active ingredient | 20 mg |
| Dried starch | 178 mg |
| Magnesium stearate | 2 mg |
| Total | 200 mg |

The active ingredient, starch and magnesium stearate are passed through a sieve and filled into hard gelatine capsules in 200 mg quantities.

## Claims

**1.** A compound of formula (I)

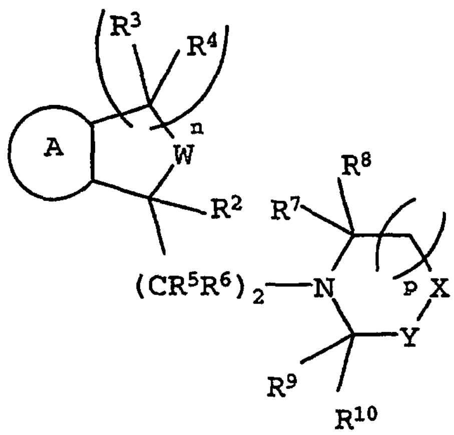

**(I)**

in which

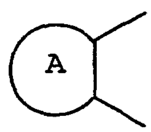

is

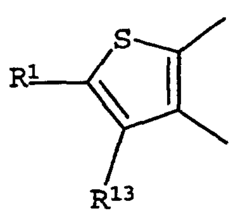

or

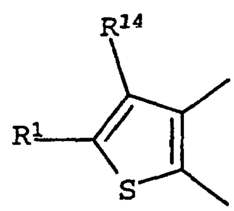

$R^1$ is -CN, $-CONR^{11}R^{12}$, $-COOR^{21}$,

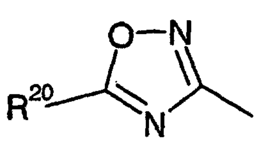

,

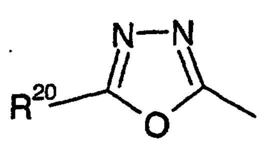

,

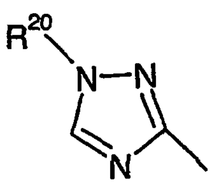

,

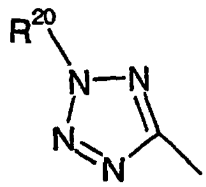

,

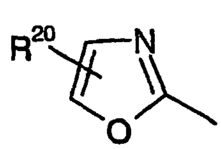

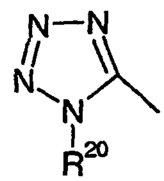 , 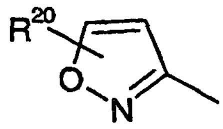 or 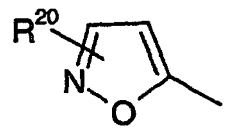

where $R^{11}$ and $R^{12}$ are each hydrogen or $C_{1-6}$ alkyl, or $R^{11}$ and $R^{12}$ taken together with the nitrogen atom to which they are attached form a morpholino, pyrrolidino or piperidinyl ring optionally substituted with one or two $C_{1-6}$ alkyl groups;
$R^2$, $R^3$, $R^4$, $R^5$, $R^6$, $R^7$, $R^8$, $R^9$, $R^{10}$, $R^{20}$ and $R^{21}$ are each hydrogen or $C_{1-6}$ alkyl;
$R^{13}$ and $R^{14}$ are each independently selected from hydrogen, $C_{1-6}$ alkyl or halo;
n is 1 or 2;
p is 0, 1 or 2;
-W- is -O- or $-CF_2-$;
-X-Y- is

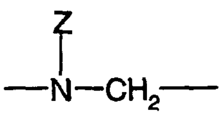 , 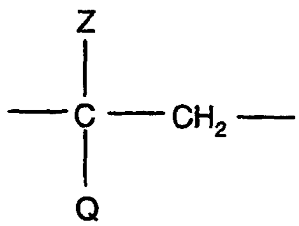 or 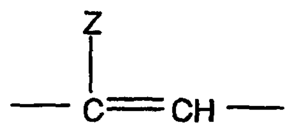

where Z is

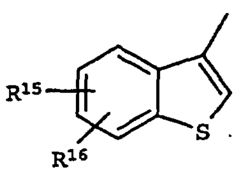
(i)

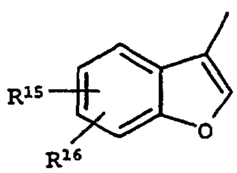
(ii)

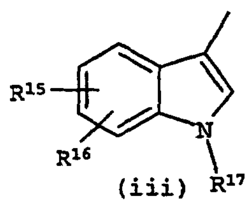
(iii)

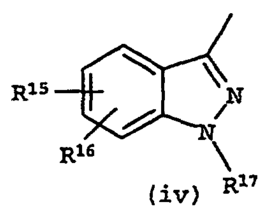
(iv)

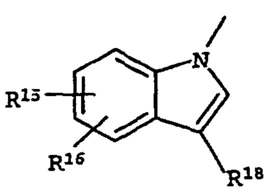
(v)

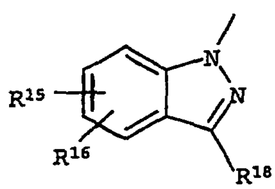
(vi)

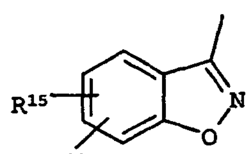
(vii)

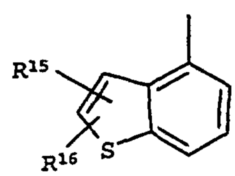
(viii)

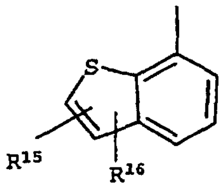
(ix)

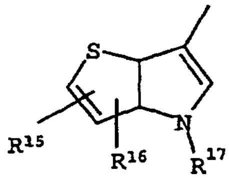
(x)

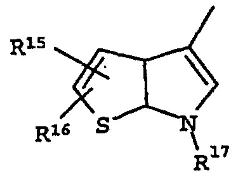
(xi)

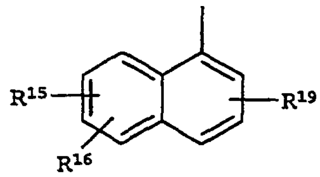
(xii)

where $R^{15}$, $R^{16}$ and $R^{19}$ are each hydrogen, halo, $C_{1-6}$ alkyl or $C_{1-6}$ alkoxy, $C_{1-6}$ alkoxycarbonyl, cyano, trifluoromethyl, trifluoromethoxy, nitro, amino, $C_1$-$C_6$ acylamino and $C_1$-$C_6$ alkylthio; and
$R^{17}$ and $R^{18}$ are hydrogen or $C_{1-6}$ alkyl;
Q is hydrogen, halo, nitrile, $C_{1-6}$ alkoxycarbonyl, hydroxy, $C_{1-6}$ alkyl or $C_{1-6}$ alkoxy;
and pharmaceutically acceptable salts thereof.

**2.** A compound as claimed in Claim 1 wherein n is 2.

**3.** A compound as claimed in Claim 1 or Claim 2 wherein $R^1$ is -CN.

**4.** A compound as claimed in Claim 1 or Claim 2 wherein $R^1$ is $-COOR^{21}$.

**5.** A compound as claimed in Claim 1 or claim 2 wherein $R^1$ is $-CONR^{11}R^{12}$.

**6.** A compound as claimed in Claim 4 wherein $R^{21}$ is $C_{1-6}$ alkyl.

**7.** A compound as claimed in Claim 6 wherein $R^{21}$ is methyl.

**8.** A compound as claimed in Claim 5 wherein $R^{11}$ and $R^{12}$ are both hydrogen.

**9.** A compound as claimed in any one of the preceding claims wherein

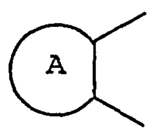

is

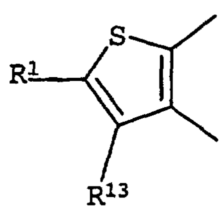

**10.** A compound as claimed in any one of claims 1 to 8 wherein

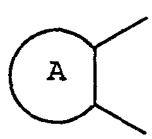

is

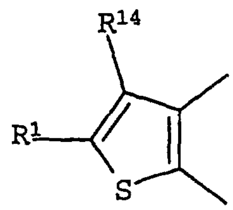

11. A compound as claimed in Claim 9 wherein $R^{13}$ is hydrogen.

12. A compound as claimed in Claim 10 wherein $R^{14}$ is hydrogen.

13. A compound as claimed in Claim 10 wherein $R^{14}$ is halo.

14. A compound as claimed in Claim 10 wherein $R^{14}$ is $C_{1-6}$ alkyl.

15. A compound as claimed in any one of the preceding claims wherein $R^2$ is hydrogen or $C_{1-6}$ alkyl.

16. A compound as claimed in Claim 15 wherein $R^2$ is hydrogen or methyl.

17. A compound as claimed in Claim 16 wherein $R^2$ is hydrogen.

18. A compound as claimed in any one of the preceding claims wherein $R^3$ to $R^6$ are each hydrogen.

19. A compound as claimed in any one of the preceding claims wherein $R^7$ to $R^{10}$ are each $C_{1-6}$ alkyl or hydrogen.

20. A compound as claimed in Claim 19 wherein one of $R^7$ and $R^8$ or one of $R^9$ and $R^{10}$ is $C_{1-6}$ alkyl and the remainder are each hydrogen.

21. A compound as claimed in any one of the preceding claims wherein p is 1.

22. A compound as claimed in any one of the preceding claims wherein -W- is -O-.

23. A compound as claimed in any one of Claims 1 to 21 wherein -W- is $-CF_2-$.

24. A compound as claimed in any one of the preceding Claims wherein -X-Y- is

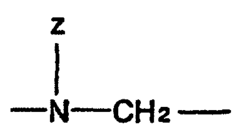

25. A compound as claimed in any one of claims 1 to 23 wherein -X-Y- is

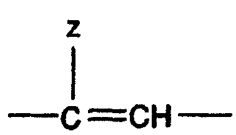

26. A compound as claimed in any one of the preceding claims wherein Z is

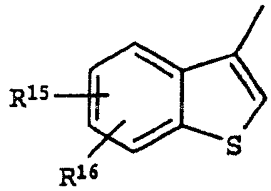

(i)

27. A compound as claimed in any one of Claims 1 to 25 wherein Z is

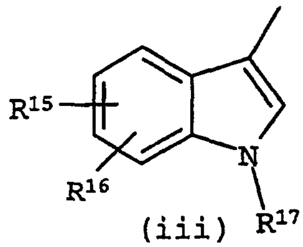

(iii)

28. A compound as claimed in any one of Claims 1 to 25 wherein Z is

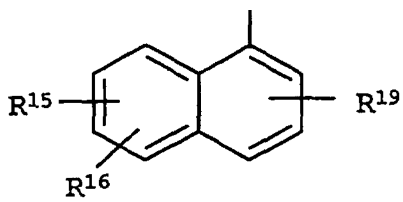

(xii)

29. A compound as claimed in any one of Claims 26 to 28 wherein $R^{15}$, $R^{16}$, and $R^{19}$, where present, are each hydrogen, halo or $C_{1-6}$ alkoxy.

30. A compound as claimed in Claim 29 wherein one of $R^{15}$, $R^{16}$ and $R^{19}$ is halo or $C_{1-6}$ alkoxy and the remainder are each hydrogen.

31. A compound as claimed in any one of Claims 26 to 28 wherein $R^{15}$ is fluoro, hydrogen or methoxy.

32. A compound as claimed in any one of Claims 26 to 28 wherein $R^{16}$ is fluoro, hydrogen or methoxy.

33. A compound as claimed in Claim 28 wherein $R^{19}$ is fluoro or hydrogen.

34. A compound as claimed in Claim 27 wherein $R^{17}$ is hydrogen.

35. A pharmaceutical formulation comprising a compound of formula I as claimed in any one of Claims 1 to 34 or a pharmaceutically acceptable salt thereof, together with a pharmaceutically acceptable carrier, diluent or excipient.

36. A process for preparing a compound of formula I as claimed in any one of Claims 1 to 34, or a salt or ester thereof, which comprises reacting a compound having the formula:

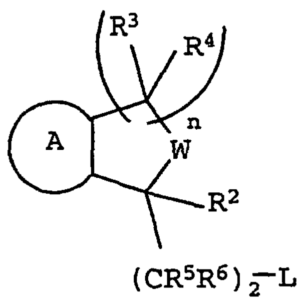

(III)

where L is a leaving group, with a compound of the formula:

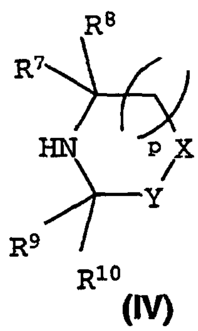

(IV)

wherein $R^2$ to $R^{10}$, -X-Y-, A, -W-, n and p have the values defined in claim 1.

**37.** A compound of formula I as claimed in any one of Claims 1 to 34, or a pharmaceutically acceptable salt thereof, for use as a pharmaceutical.

**38.** Use of a compound of formula I as claimed in any one of Claims 1 to 34, or a pharmaceutically acceptable ester thereof, or a pharmaceutically acceptable salt thereof, for the manufacture of a medicament for treating a disorder of the central nervous system.

**39.** Use as claimed in Claim 38, wherein the disorder is depression.

**40.** Use as claimed in Claim 38, wherein the disorder is anxiety.

**41.** Use as claimed in Claim 38, wherein the disorder is a disorder with depressive or anxiety symptoms.

**Patentansprüche**

**1.** Verbindung der Formel (I)

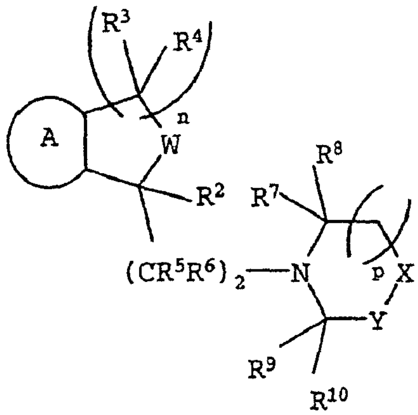

**(I)**

worin

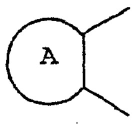

steht für

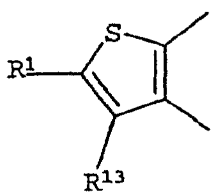

oder

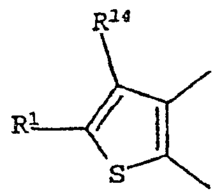

$R^1$ steht für

-CN, $-CONR^{11}R^{12}$, $-COOR^{21}$,

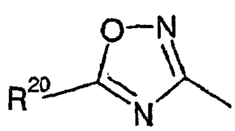

,

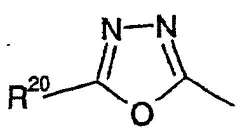

,

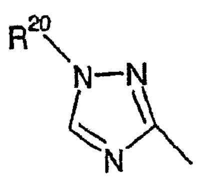 , 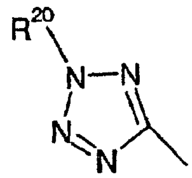 , 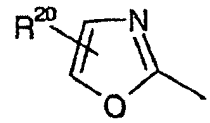

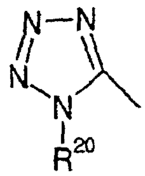 , 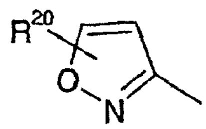 oder 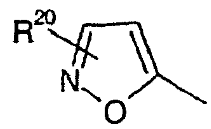

worin $R^{11}$ und $R^{12}$ jeweils für Wasserstoff oder $C_1$-$C_6$ Alkyl stehen oder $R^{11}$ und $R^{12}$ zusammengenommen mit dem Stickstoffatom, an das sie gebunden sind, einen Morpholino-, Pyrrolidino- oder Piperidinylring bilden, der wahlweise mit ein oder zwei $C_1$-$C_6$ Alkylgruppen substituiert sein kann,
$R^2$, $R^3$, $R^4$, $R^5$, $R^6$, $R^7$, $R^8$, $R^9$, $R^{10}$, $R^{20}$ und $R^{21}$ jeweils für Wasserstoff oder $C_1$-$C_6$ Alkyl stehen,
$R^{13}$ und $R^{14}$ jeweils unabhängig ausgewählt sind aus Wasserstoff, $C_1$-$C_6$ Alkyl oder Halogen,
n für 1 oder 2 steht,
p für 0, 1 oder 2 steht,
-W- für -O- oder $-CF_2-$ steht,
-X-Y- steht für

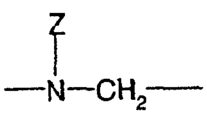 , 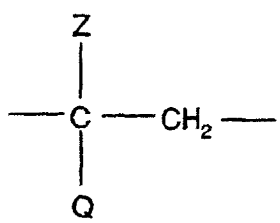 oder 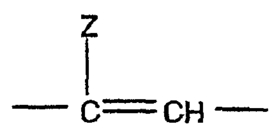

worin Z steht für

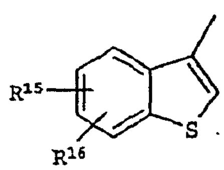
(i)

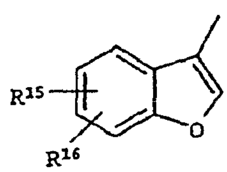
(ii)

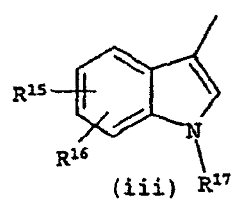
(iii)

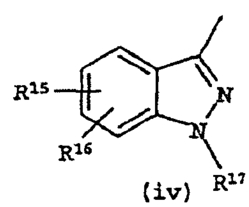
(iv)

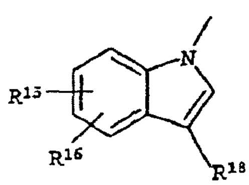
(v)

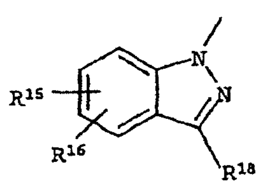
(vi)

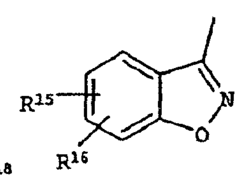
(vii)

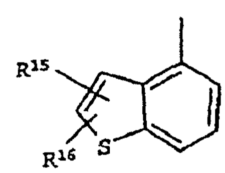
(viii)

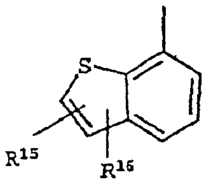
(ix)

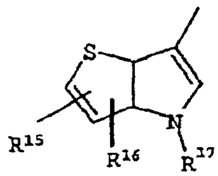
(x)

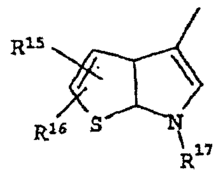
(xi)

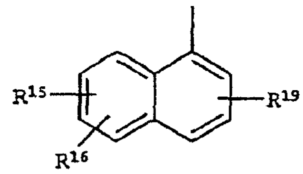
(xii)

worin $R^{15}$, $R^{16}$ und $R^{19}$ jeweils für Wasserstoff, Halogen, $C_1$-$C_6$ Alkyl oder $C_1$-$C_6$ Alkoxy, $C_1$-$C_6$ Alkoxycarbonyl, Cyano, Trifluormethyl, Trifluormethoxy, Nitro, Amino, $C_1$-$C_6$ Acylamino und $C_1$-$C_6$ Alkylthio stehen, und
$R^{17}$ und $R^{18}$ für Wasserstoff oder $C_1$-$C_6$ Alkyl stehen,
Q für Wasserstoff, Halogen, Nitril, $C_1$-$C_6$ Alkoxycarbonyl, Hydroxy, $C_1$-$C_6$ Alkyl oder $C_1$-$C_6$ Alkoxy steht, und pharmazeutisch annehmbare Salze hiervon.

**2.** Verbindung nach Anspruch 1, worin n für 2 steht.

**3.** Verbindung nach Anspruch 1 oder Anspruch 2, worin $R^1$ für -CN steht.

**4.** Verbindung nach Anspruch 1 oder Anspruch 2, worin $R^1$ für -$COOR^{21}$ steht.

**5.** Verbindung nach Anspruch 1 oder Anspruch 2, worin $R^1$ für -$CONR^{11}R^{12}$ steht.

**6.** Verbindung nach Anspruch 4, worin $R^{21}$ für $C_1$-$C_6$ Alkyl steht.

**7.** Verbindung nach Anspruch 6, worin $R^{21}$ für Methyl steht.

**8.** Verbindung nach Anspruch 5, worin $R^{11}$ und $R^{12}$ beide für Wasserstoff stehen.

**9.** Verbindung nach einem der vorangehenden Ansprüche, worin

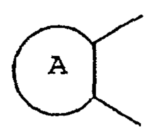

steht für

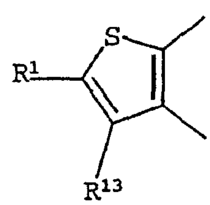

**10.** Verbindung nach einem der Ansprüche 1 bis 8, worin

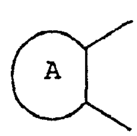

steht für

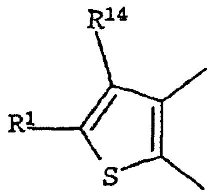

**11.** Verbindung nach Anspruch 9, worin $R^{13}$ für Wasserstoff steht.

**12.** Verbindung nach Anspruch 10, worin $R^{14}$ für Wasserstoff steht.

**13.** Verbindung nach Anspruch 10, worin $R^{14}$ für Halogen steht.

**14.** Verbindung nach Anspruch 10, worin $R^{14}$ für $C_1$-$C_6$ Alkyl steht.

**15.** Verbindung nach einem der vorangehenden Ansprüche, worin $R^2$ für Wasserstoff oder $C_1$-$C_6$ Alkyl steht.

**16.** Verbindung nach Anspruch 15, worin $R^2$ für Wasserstoff oder Methyl steht.

**17.** Verbindung nach Anspruch 16, worin $R^2$ für Wasserstoff steht.

**18.** Verbindung nach einem der vorangehenden Ansprüche, worin $R^3$ bis $R^6$ jeweils für Wasserstoff stehen.

**19.** Verbindung nach einem der vorangehenden Ansprüche, worin $R^7$ bis $R^{10}$ jeweils für $C_1$-$C_6$ Alkyl oder Wasserstoff stehen.

**20.** Verbindung nach Anspruch 19, worin eines von $R^7$ und $R^8$ oder eines von $R^9$ und $R^{10}$ für $C_1$-$C_6$ Alkyl steht und die anderen jeweils für Wasserstoff stehen.

**21.** Verbindung nach einem der vorangehenden Ansprüche, worin p für 1 steht.

**22.** Verbindung nach einem der vorangehenden Ansprüche, worin -W- für -O- steht.

**23.** Verbindung nach einem der Ansprüche 1 bis 21, worin -W- für $-CF_2-$ steht.

**24.** Verbindung nach einem der vorangehenden Ansprüche, worin -X-Y- steht für

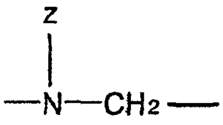

**25.** Verbindung nach einem der Ansprüche 1 bis 23, worin -X-Y- steht für

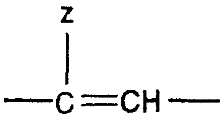

**26.** Verbindung nach einem der vorangehenden Ansprüche, worin Z steht für

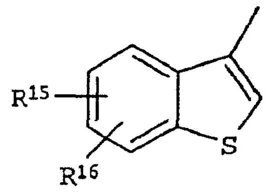

(i)

**27.** Verbindung nach einem der Ansprüche 1 bis 25, worin Z steht für

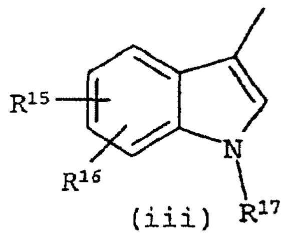

(iii)

**28.** Verbindung nach einem der Ansprüche 1 bis 25, worin Z steht für

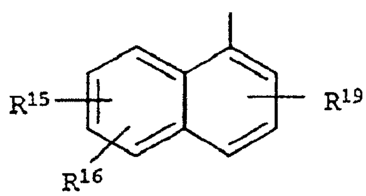

(xii)

**29.** Verbindung nach einem der Ansprüche 26 bis 28, worin $R^{15}$, $R^{16}$ und $R^{19}$, wenn sie vorkommen, jeweils für Wasserstoff, Halogen oder $C_1$-$C_6$ Alkoxy stehen.

**30.** Verbindung nach Anspruch 29, worin eines von $R^{15}$, $R^{16}$ und $R^{19}$ für Halogen oder $C_1$-$C_6$ Alkoxy steht und die anderen jeweils für Wasserstoff stehen.

**31.** Verbindung nach einem der Ansprüche 26 bis 28, worin $R^{15}$ für Fluor, Wasserstoff oder Methoxy steht.

**32.** Verbindung nach einem der Ansprüche 26 bis 28, worin $R^{16}$ für Fluor, Wasserstoff oder Methoxy steht.

**33.** Verbindung nach Anspruch 28, worin $R^{19}$ für Fluor oder Wasserstoff steht.

**34.** Verbindung nach Anspruch 27, worin $R^{17}$ für Wasserstoff steht.

**35.** Pharmazeutische Formulierung, die eine Verbindung der Formel I nach einem der Ansprüche 1 bis 34 oder ein pharmazeutisch annehmbares Salz hiervon zusammen mit einem pharmazeutisch annehmbaren Träger, Verdünnungsmittel oder Hilfsstoff enthält.

**36.** Verfahren zur Herstellung einer Verbindung der Formel I nach einem der Ansprüche 1 bis 34 oder eines Salzes oder Esters hiervon, **gekennzeichnet durch** Umsetzung einer Verbindung der Formel

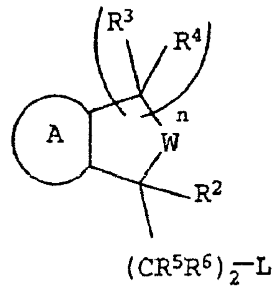

(III)

worin L für eine Abgangsgruppe steht, mit einer Verbindung der Formel

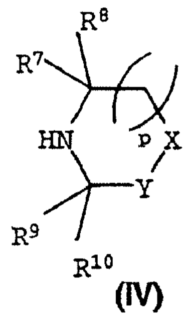

(IV)

worin $R^2$ bis $R^{10}$, -X-Y-, A, -W-, n und p die in Anspruch 1 definierten Bedeutungen haben.

**37.** Verbindung der Formel I nach einem der Ansprüche 1 bis 34 oder ein pharmazeutisch annehmbares Salz hiervon zur Verwendung als Pharmazeutikum.

**38.** Verwendung einer Verbindung der Formel I nach einem der Ansprüche 1 bis 34 oder eines pharmazeutisch annehmbaren Esters hiervon oder eines pharmazeutisch annehmbaren Salzes hiervon zur Herstellung eines Arzneimittels zur Behandlung einer Störung des zentralen Nervensystems.

**39.** Verwendung nach Anspruch 38, worin die Störung Depression ist.

**40.** Verwendung nach Anspruch 38, worin die Störung Angst ist.

**41.** Verwendung nach Anspruch 38, worin die Störung eine Störung mit Depressions- oder Angstsymptomen ist.

**Revendications**

**1.** Composé répondant à la formule (I)

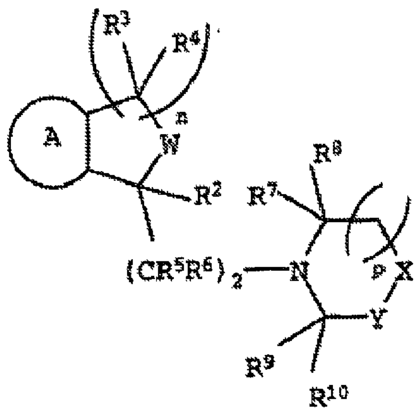

(I)

dans laquelle

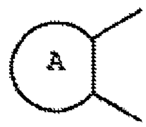

représente

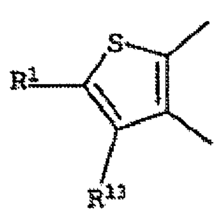

ou

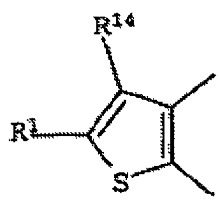

;

$R^1$ représente
-CN, $-CONR^{11}R^{12}$, $-COOR^{21}$,

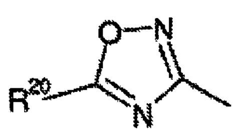

,

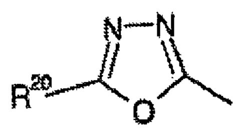

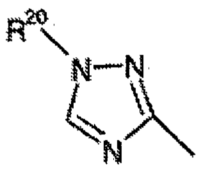 , 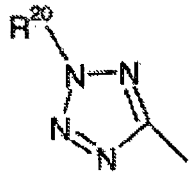 , 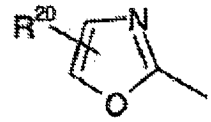

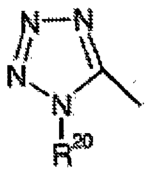 , 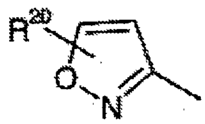 ou 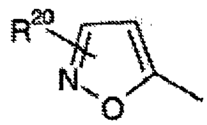 ;

où $R^{11}$ et $R^{12}$ représentent chacun un atome d'hydrogène ou un groupe alkyle en $C_1$-$C_6$ ou $R^{11}$ et $R^{12}$ pris ensemble avec l'atome d'azote auquel ils sont attachés forment un cycle morpholino, pyrrolidino ou pipéridinyle éventuellement substitué par un ou deux groupes alkyles en $C_1$-$C_6$ ;
$R^2$, $R^3$, $R^4$, $R^5$, $R^6$, $R^7$, $R^8$, $R^9$, $R^{10}$, $R^{20}$ et $R^{21}$ représentent chacun un atome d'hydrogène ou un groupe alkyle en $C_1$-$C_6$ ;
$R^{13}$ et $R^{14}$ sont chacun indépendamment choisis parmi un atome d'hydrogène, un groupe alkyle en $C_1$-$C_6$ ou un atome d'halogène ;
n vaut 1 ou 2 ;
p vaut 0, 1 ou 2 ;
-W- représente -O- ou $-CF_2-$ ;
-X-Y- représente

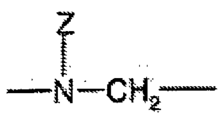 , 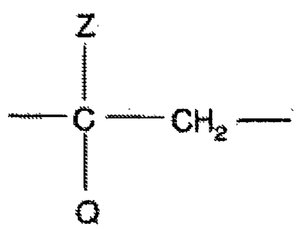 ou 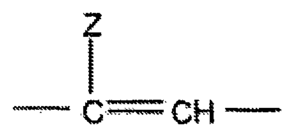

où Z représente

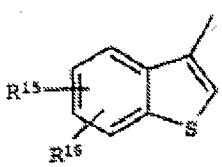
(i)

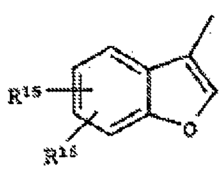
(ii)

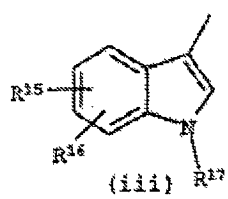
(iii)

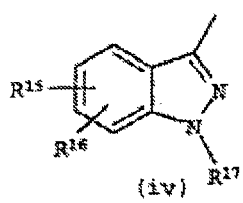
(iv)

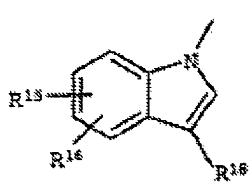
(v)

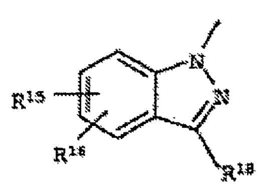
(vi)

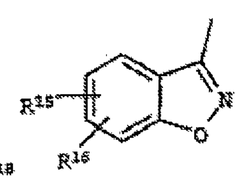
(vii)

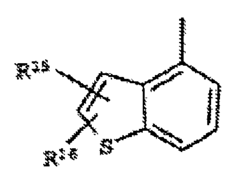
(viii)

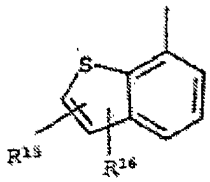

(ix)

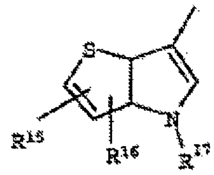

(x)

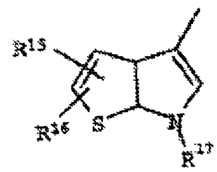

(xi)

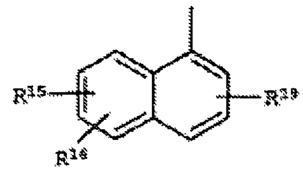

(xii)

où $R^{15}$, $R^{16}$ et $R^{19}$ représentent chacun un atome d'hydrogène, un atome d'halogène, un groupe alkyle en $C_1$-$C_6$ ou un groupe alcoxy en $C_1$-$C_6$, un groupe (alcoxy en $C_1$-$C_6$)carbonyle, un groupe cyano, un groupe trifluorométhyle, un groupe trifluorométhoxy, un groupe nitro, un groupe amino, un groupe acylamino en $C_1$-$C_6$ et un groupe alkylthio en $C_1$-$C_6$ ; et
$R^{17}$ et $R^{18}$ représentent un atome d'hydrogène ou un groupe alkyle en $C_1$-$C_6$ ;
Q représente un atome d'hydrogène, un atome d'halogène, un groupe nitrile, un groupe (alcoxy en $C_1$-$C_6$)carbonyle, un groupe hydroxy, un groupe alkyle en $C_1$-$C_6$ ou un groupe alcoxy en $C_1$-$C_6$ ;
et des sels pharmaceutiquement acceptables de celui-ci.

**2.** Composé selon la revendication 1, dans lequel n vaut 2.

**3.** Composé selon la revendication 1 ou 2, dans lequel $R^1$ représente -CN.

**4.** Composé selon la revendication 1 ou 2, dans lequel $R^1$ représente $-COOR^{21}$.

**5.** Composé selon la revendication 1 ou 2, dans lequel $R^1$ représente $-CONR^{11}R^{12}$.

**6.** Composé selon la revendication 4, dans lequel $R^{21}$ représente un groupe alkyle en $C_1$-$C_6$.

**7.** Composé selon la revendication 6, dans lequel $R^{21}$ représente un groupe méthyle.

**8.** Composé selon la revendication 5, dans lequel $R^{11}$ et $R^{12}$ représentent tous deux un atome d'hydrogène.

**9.** Composé selon l'une quelconque des revendications précédentes, dans lequel

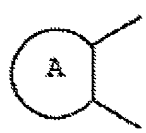

représente

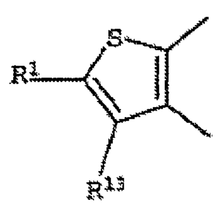

.

**10.** Composé selon l'une quelconque des revendications 1 à 8, dans lequel

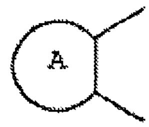

représente

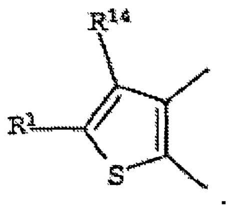

**11.** Composé selon la revendication 9, dans lequel $R^{13}$ représente un atome d'hydrogène.

**12.** Composé selon la revendication 10, dans lequel $R^{14}$ représente un atome d'hydrogène.

**13.** Composé selon la revendication 10, dans lequel $R^{14}$ représente un atome d'halogène.

**14.** Composé selon la revendication 10, dans lequel $R^{14}$ représente un groupe alkyle en $C_1$-$C_6$.

**15.** Composé selon l'une quelconque des revendications précédentes, dans lequel $R^2$ représente un atome d'hydrogène ou un groupe alkyle en $C_1$-$C_6$.

**16.** Composé selon la revendication 15, dans lequel $R^2$ représente un atome d'hydrogène ou un groupe méthyle.

**17.** Composé selon la revendication 16, dans lequel $R^2$ représente un atome d'hydrogène.

**18.** Composé selon l'une quelconque des revendications précédentes, dans lequel $R^3$ à $R^6$ représentent chacun un atome d'hydrogène.

**19.** Composé selon l'une quelconque des revendications précédentes, dans lequel $R^7$ à $R^{10}$ représentent chacun un groupe alkyle en $C_1$-$C_6$ ou un atome d'hydrogène.

**20.** Composé selon la revendication 19, dans lequel un de $R^7$ et $R^8$ ou un de $R^9$ et $R^{10}$ représente un groupe alkyle en $C_1$-$C_6$ et les autres représentent chacun un atome d'hydrogène.

**21.** Composé selon l'une quelconque des revendications précédentes, dans lequel p vaut 1.

**22.** Composé selon l'une quelconque des revendications précédentes, dans lequel -W- représente -O-.

**23.** Composé selon l'une quelconque des revendications 1 à 21, dans lequel - W- représente $-CF_2-$.

**24.** Composé selon l'une quelconque des revendications précédentes, dans lequel -X-Y- représente

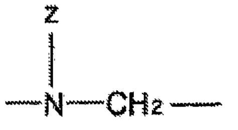

**25.** Composé selon l'une quelconque des revendications 1 à 23, dans lequel -X-Y- représente

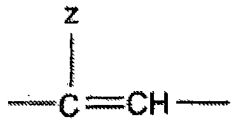

**26.** Composé selon l'une quelconque des revendications précédentes, dans lequel Z représente

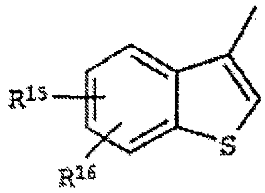

(i)

.

**27.** Composé selon l'une quelconque des revendications 1 à 25, dans lequel Z représente

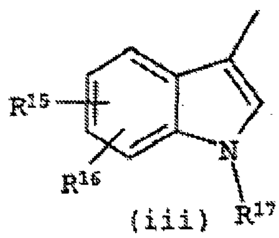

(iii)

.

**28.** Composé selon l'une quelconque des revendications 1 à 25, dans lequel Z représente

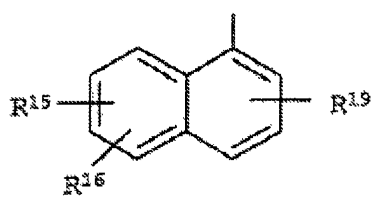

(xii)

.

**29.** Composé selon l'une quelconque des revendications 26 à 28, dans lequel $R^{15}$, $R^{16}$ et $R^{19}$, lorsqu'ils sont présents, représentent chacun un atome d'hydrogène, un atome d'halogène ou un groupe alcoxy en $C_1$-$C_6$.

**30.** Composé selon la revendication 29, dans lequel un de $R^{15}$, $R^{16}$ et $R^{19}$ représente un atome d'halogène ou un groupe alcoxy en $C_1$-$C_6$ et les autres représentent chacun un atome d'hydrogène.

**31.** Composé selon l'une quelconque des revendications 26 à 28, dans lequel $R^{15}$ représente un atome de fluor, un atome d'hydrogène ou un groupe méthoxy.

**32.** Composé selon l'une quelconque des revendications 26 à 28, dans lequel $R^{16}$ représente un atome de fluor, un atome d'hydrogène ou un groupe méthoxy.

**33.** Composé selon la revendication 28, dans lequel $R^{19}$ représente un atome de fluor ou un atome d'hydrogène.

**34.** Composé selon la revendication 27, dans lequel $R^{17}$ représente un atome d'hydrogène.

**35.** Formulation pharmaceutique comprenant un composé répondant à la formule I selon l'une quelconque des revendications 1 à 34 ou un sel pharmaceutiquement acceptable de celui-ci, avec un support, un diluent ou un excipient pharmaceutiquement acceptable.

**36.** Procédé de préparation d'un composé répondant à la formule I selon l'une quelconque des revendications 1 à 34 ou d'un sel ou d'un ester de celui-ci, qui comprend la réaction d'un composé répondant à la formule

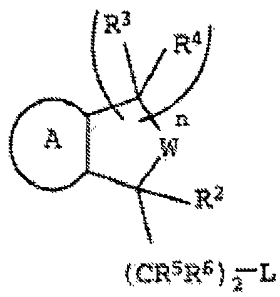

**(III)**

où L est un groupe sortant, avec un composé répondant à la formule

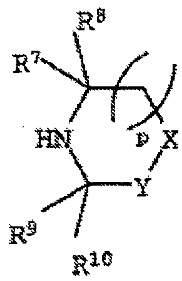

**(IV)**

où $R^2$ à $R^{10}$, -X-Y-, A, -W-, n et p ont les significations données dans la revendication 1.

**37.** Composé répondant à la formule 1 selon l'une quelconque des revendications 1 à 34, ou un sel pharmaceutiquement acceptable de celui-ci, pour une utilisation comme produit pharmaceutique.

**38.** Utilisation d'un composé répondant à la formule I selon l'une quelconque des revendications 1 à 34 ou d'un ester pharmaceutiquement acceptable de celui-ci, ou d'un sel pharmaceutiquement acceptable de celui-ci, pour la fabrication d'un médicament destiné à traiter un trouble du système nerveux central.

**39.** Utilisation selon la revendication 38, dans laquelle le trouble est une dépression.

**40.** Utilisation selon la revendication 38, dans laquelle le trouble est une anxiété.

**41.** Utilisation selon la revendication 38, dans laquelle le trouble est un trouble présentant des symptômes de dépression ou d'anxiété.